# FASCICULE DE BREVET EUROPEEN

(11) **EP 2 563 807 B1**
(45) Date de publication et mention de la délivrance du brevet: **19.10.2016**
(21) Numéro de dépôt: 11723513.5
(22) Date de dépôt: 27.04.2011
(51) Int. Cl.: C07K 14/47, A61K 8/64, A61K 38/00, A61Q 19/00, A61Q 19/08, C07K 7/06, C07K 7/08

(54) **POLYPEPTIDE EXPRIMÉ DANS LA COUCHE CORNÉE ET SON UTILISATION**
IM STRATUM CORNEUM EXPRIMIERTES POLYPEPTID UND VERWENDUNG DAVON
POLYPEPTIDE EXPRESSED IN THE STRATUM CORNEUM AND USE THEREOF

(30) Priorité: 27.04.2010 FR 1053225
(43) Date de publication de la demande: 06.03.2013
(73) Titulaire: Centre National de la Recherche Scientifique, 75016 Paris (FR); Université Paul Sabatier (Toulouse III), 31400 Toulouse (FR)
(72) Inventeur: JONCA, Nathalie, F-31870 Lagardelle Sur Leze (FR); TOULZA, Eve, F-66300 Nyls Ponteilla (FR); SAINTIGNY, Gaëlle, F-75019 Paris (FR); SERRE, Guy, F-31100 Toulouse (FR); WEBER VIVAT, Marina, F-11320 Saint Paulet (FR)
(74) Mandataire: Cabinet Plasseraud
(86) Numéro de dépôt international: PCT/FR2011/050953
(87) Numéro de publication internationale: WO 2011/135253

(56) Documents cités:
- EP-A1- 1 342 474
- WO-A1-96/40780
- WO-A2-02/07707
- WO-A2-02/059260
- WO-A2-2008/016356
- DATABASE UniProt [Online] 14 novembre 2006 (2006-11-14), "RecName: Full=Late cornified envelope protein 6A;", XP002604655, extrait de EBI accession no. UNIPROT:A0A183 Database accession no. A0A183
- DATABASE EMBL [Online] 20 octobre 2006 (2006-10-20), "Homo sapiens late cornified envelope 6A (LCE6A) mRNA, complete cds.", XP002604656, extrait de EBI accession no. EMBL:DQ991251 Database accession no. DQ991251
- MARSHALL D ET AL: "Differentially expressed late constituents of the epidermal cornified envelope", PROCEEDINGS OF THE NATIONAL ACADEMY OF SCIENCES OF THE UNITED STATES OF AMERICA, vol. 98, no. 23, 2001, pages 13031-13036, XP002604657, ISSN: 0027-8424
- DE CID RAFAEL ET AL: "Deletion of the late cornified envelope LCE3B and LCE3C genes as a susceptibility factor for psoriasis", NATURE GENETICS, vol. 41, no. 2, février 2009 (2009-02), pages 211-215, XP002604658, ISSN: 1061-4036

### SEQUENCE LISTING

<110> Chanel parfums Beauté et Centre National de la Recherche Scientifique
<120> Polypeptide exprimé dans la couche cornée et son utilisation
<130> BFF100137
<160> 8
<170> PatentIn version 3.5
<210> 1
   <211> 641
   <212> DNA
   <213> Homo sapiens
<400> 1
   acacaaggcc tccagactcc tcctgggata acttgtccac acatcttcca ctagggtaag        60
   gctacttctg gctgaggaga cacttggatg tagctcaagt gctgcttagg cagtcctgat       120
   ctctcctctc gtctcttccc agggagctga aaagccagat tcgacctggt agccaagcaa       180
   tgtcacagca gaagcagcaa tcttggaagc ctccaaatgt tcccaaatgc tcccctcccc       240
   aaagatcaaa cccctgccta gctccctact cgactccttg tggtgctccc cattcagaag       300
   gttgtcattc cagttcccaa aggcctgagg ttcagaagcc taggagggct cgtcaaaagc       360
   tgcgctgcct aagtaggggc acaacctacc actgcaaaga ggaagagtgt gaaggcgact       420
   gagcccagaa gagttgaggc acaggtgcag ttactctctc cctgccccac ctttgggtac       480
   taattccccc ttggaaagcc aggccctcaa cctctcattt ggactgagaa acacttcctg       540
   atccccagct ctagagaagc gagaactagg ctgagccacg ctgctactgc tctcttccat       600
   tcaccccttc agctcagcaa caataaagct gctttacttg g                           641
<210> 2
   <211> 80
   <212> PRT
   <213> Homo sapiens
<400> 2
   Met Ser Gln Gln Lys Gln Gln Ser Trp Lys Pro Pro Asn Val Pro Lys
   1               5                   10                  15
   Cys Ser Pro Pro Gln Arg Ser Asn Pro Cys Leu Ala Pro Tyr Ser Thr
               20                  25                  30
   Pro Cys Gly Ala Pro His Ser Glu Gly Cys His Ser Ser Ser Gln Arg
           35                  40                  45
   Pro Glu Val Gln Lys Pro Arg Arg Ala Arg Gln Lys Leu Arg Cys Leu
       50                  55                  60
   Ser Arg Gly Thr Thr Tyr His Cys Lys Glu Glu Glu Cys Glu Gly Asp
   65                  70                  75                  80
<210> 3
   <211> 9
   <212> PRT
   <213> Artificial
<220>
   <223> Designed peptide based on homology to amino acid residue 1 to 9 from Protein LCE6A
<400> 3
   Met Ser Gln Gln Lys Gln Gln Ser Trp
   1               5
<210> 4
   <211> 11
   <212> PRT
   <213> Artificial
<220>
   <223> Designed peptide based on homology to amino acid residue 45 to 55 from Protein LCE6A
<400> 4
   Ser Ser Gln Arg Pro Glu Val Gln Lys Pro Arg
   1               5                   10
<210> 5
   <211> 13
   <212> PRT
   <213> Artificial
<220>
   <223> Designed peptide based on homology to amino acid residue 50 to 62 from Protein LCE6A
<400> 5
   Glu Val Gln Lys Pro Arg Arg Ala Arg Gln Lys Leu Arg
   1               5                   10
<210> 6
   <211> 15
   <212> PRT
   <213> Artificial
<220>
   <223> Designed peptide based on homology to amino acid residue 42 to 56 from Protein LCE6A
<400> 6
   Cys His Ser Ser Ser Gln Arg Pro Glu Val Gln Lys Pro Arg Arg
   1               5                   10                  15
<210> 7
   <211> 9
   <212> PRT
   <213> Artificial Sequence
<220>
   <223> Mutated peptide of SEQ ID NO°3
<400> 7
   Met Ser Gln Ala Lys Gln Gln Ser Trp
   1 5
<210> 8
   <211> 13
   <212> PRT
   <213> Artificial Sequence
<220>
   <223> Mutated peptide of SEQ ID N°5
<400> 8
   Glu Val Ala Lys Pro Arg Arg Ala Arg Gln Ala Leu Arg
   1 5 10

## Description

La présente invention concerne un nouveau polypeptide de la couche cornée, ci-après dénommé LCE6A, ainsi que ses applications, tant au niveau cosmétique que thérapeutique.

La peau est principalement constituée de trois couches, à savoir, en partant de la plus superficielle, l'épiderme, le derme et l'hypoderme.

L'épiderme est en particulier constitué de kératinocytes (majoritaires), de mélanocytes (intervenant dans la pigmentation de la peau) et de cellules de Langerhans. Sa fonction est de protéger le corps de l'environnement extérieur et d'assurer son intégrité, et notamment de freiner la pénétration de micro-organismes ou de substances chimiques, et d'empêcher l'évaporation de l'eau contenue dans la peau.

Pour ce faire, les kératinocytes subissent un processus de maturation orientée continu au cours duquel les kératinocytes situés dans la couche basale de l'épiderme forment, au stade terminal de leur différenciation, des cornéocytes qui sont des cellules issues de la cornification (apoptose particulière). Ces cornéocytes sont très inter-cohésifs, totalement kératinisés sous forme d'enveloppes cornées et entourés d'un milieu extracellulaire très riche en lipides. Les éléments constitutifs de ces cellules, ainsi que les enzymes qui régulent leur détachement pour permettre la desquamation, sont principalement synthétisés par les kératinocytes de la couche cellulaire sous-jacente, la couche granuleuse. Les kératinocytes granuleux correspondent au dernier stade nucléé de la différenciation kératinocytaire, avant la cornification qui s'accompagne de lyse nucléaire avec arrêt de toute activité de transcription et de traduction. C'est à ce stade que culmine la production des précurseurs de l'enveloppe cornée et autres constituants cellulaires spécifiques indispensables à la fonction barrière de l'épiderme tels que les céramides, le cholestérol et les acides gras libres. L'enveloppe cornée formée au cours de la cornification se substitue à la bicouche lipidique des kératinocytes granuleux. Elle représente 7% du poids sec de la couche cornée. Elle est constituée de protéines liées entre elles ou à l'enveloppe lipidique lamellaire de façon covalente par les transglutaminases formant un complexe macromoléculaire particulièrement stable, insoluble et imperméable, qui est essentiel à la résistance physique et à la fonction barrière de la couche cornée.

La différenciation épidermique est un phénomène complexe nécessitant une régulation fine de l'expression des gènes permettant la fabrication des différents constituants des kératinocytes puis des cornéocytes. De nombreux facteurs de transcription sont impliqués dans ce processus. Les gènes de nombreuses protéines de l'enveloppe cornée sont localisés au sein d'un même cluster de 2,5 Mb appelé « Complexe de Différenciation Epidermique » (CDE) en position 1q21.3. Le CDE comprend plus de 50 gènes différents exprimés principalement dans l'épiderme. On y retrouve la plupart des gènes codant pour les protéines structurales nécessaires à la différenciation terminale comme la loricrine, la filaggrine et l'involucrine. Le CDE contient également plusieurs familles de gènes, dont au moins 18 codent pour les protéines tardives de l'enveloppe (Late Cornified Envelope, LCE) (Marshall et al. 2001, Differentially expressed late constituents of the epidermal cornified envelope. Proc Natl Acad Sci U S A. 98:13031-6). Au cours de la cornification, ces protéines de la famille LCE sont incorporées à l'enveloppe cornée grâce à l'action des transglutaminases qui établissent un lien ε-(γ-glutamyl) lysyl entre un résidu glutamine donneur et un groupement amine accepteur, de façon dépendante du calcium.

La fonction barrière de l'épiderme peut se trouver perturbée dans certaines conditions climatiques (sous l'effet du froid et/ou du vent, par exemple) ; sous l'effet du stress ou de la fatigue ; sous l'effet de certains facteurs chimiques (pollution, rayonnements ultra-violets, alcool, savons irritants, produits de nettoyage domestique, détergents, etc.).

De nombreuses pathologies cutanées, qui se caractérisent par la production d'une couche cornée épaissie et par une desquamation anormale, c'est-à-dire par une hyperkératose, présentent également une fonction barrière altérée. L'hyperkératose peut survenir sur tout territoire anatomique cutané et dans des contextes cliniques très variés. Son substratum physiopathologique et sa cause sont variés. A titre d'exemples on peut citer: la xérose (ou sécheresse cutanée), les ichthyoses, le psoriasis, certaines lésions tumorales bénignes ou malignes, les hyperkératoses réactionnelles. A l'inverse, certaines manifestations pathologiques entraînent un amincissement de l'épiderme et en particulier de la couche cornée, se traduisant par une fragilité excessive du revêtement cutané. Celle-ci peut siéger dans divers territoires anatomiques, sa cause est variable et elle peut être constitutionnelle ou acquise. A titre d'exemples on peut citer: les troubles trophiques cutanés des membres inférieurs chez les patients porteurs de pathologies vasculaires, varices, artériopathies (diabète, artériosclérose...), les troubles trophiques cutanés dans le cadre d'un syndrome algo-dystrophique, les troubles trophiques consécutifs à une cicatrisation anormale.

La fonction barrière de l'épiderme peut se trouver également perturbée au cours du vieillissement. Ainsi, il est souvent constaté chez des sujets âgés, et notamment de plus de 50 ans, la manifestation d'une xérose ou d'une sècheresse des muqueuses, liée à une diminution de la sécrétion de sébum, à des modifications hormonales ou à des ralentissements du flux hydrique à travers l'épiderme. Ces perturbations de la barrière de l'épiderme provoquent une baisse de la quantité d'eau organisée, une désynchronisation de la synthèse ou une modification de la structure et/ou de la composition des bicouches de la couche granuleuse. Ces modifications favorisent ainsi la desquamation de la couche cornée, la pénétration d'allergènes, d'agents irritants ou de micro-organismes qui occasionnent ainsi un dessèchement cutané susceptible de générer des sensations d'inconfort telles que des tiraillements ou des rougeurs, et également d'altérer l'éclat du teint et la souplesse de la peau.

Pour prévenir ce phénomène ou le corriger, il est connu d'appliquer sur la peau des compositions cosmétiques ou pharmaceutiques renfermant des agents hygroscopiques, tels que des sucres ou des polyols, ou encore l'urée et l'acide lactique (composants du NMF, Natural Moisturizer Factor) destinés à capter l'eau présente dans la peau et à freiner ainsi son évaporation. On a également classiquement recours à des corps gras permettant de former un film occlusif sur la peau, tels que la vaseline qui contribue à freiner l'évaporation de l'eau. Par ailleurs, ces compositions incorporent fréquemment des actifs agissant sur une ou plusieurs des différentes cibles biologiques intervenant soit dans les processus de régénération de la peau, en particulier dans la différenciation des kératinocytes, la synthèse des lipides épidermiques et la cohésion des cornéocytes, soit dans la synthèse endogène de constituants du facteur d'hydratation naturel (NMF) de la peau, en particulier dans la synthèse de protéoglycanes.

Toutefois, il subsiste toujours le besoin de proposer de nouveaux actifs cosmétiques ou pharmaceutiques permettant de lutter plus efficacement contre le dessèchement cutané, les troubles de la fonction barrière et/ou la fragilisation de l'épiderme.

Or, la Demanderesse, suite à l'analyse du transcriptome du kératinocyte granuleux humain qu'elle a réalisée (Toulza et al., Large-scale identification of human genes implicated in epidermal barrier function, Genome Biology 2007, 8 :R107), a mis en évidence un nouveau gène situé dans le CDE sur le chromosome humain 1q21, codant pour un polypeptide de 80 acides aminés, qu'elle a choisi de nommer LCE6A, et dont elle a isolé et cloné l'ADN complémentaire (Human Gene Nomenclature, GenBank DQ991251).

La séquence du gène correspondant à cette nouvelle protéine LCE6A, d'une longueur de 1130 kb, permet d'affirmer qu'il appartient à la famille «Late Cornified Envelope» (LCE), une famille de gènes codant pour des protéines de l'enveloppe cornée. L'expression de ce gène est très fortement induite dans la fraction enrichie en kératinocytes granuleux, qui correspond à un stade tardif de la différenciation kératinocytaire. Bien qu'appartenant à la famille LCE, la séquence polypeptidique correspondant à LCE6A présente peu d'homologie avec les séquences des autres protéines de cette même famille et constitue un nouveau groupe parmi les 5 groupes connus à ce jour dans la famille LCE.

La séquence polypeptidique de la protéine LCE6A est de 80 acides aminés. Elle contient 8 résidus glutamine et 6 résidus lysine. Parmi un panel de 17 ADN complémentaires humains de tissus ou organes sains (coeur, cerveau, placenta, poumon, foie, muscle, rein, pancréas, rate, thymus, prostate, testicule, ovaire, intestin grêle, colon, leucocytes, épiderme), l'expression du gène LCE6A a été uniquement retrouvée dans l'épiderme.

Le document WO02/059260 décrit un polypeptide de séquence SEQ ID NO :2.

Selon un aspect, la présente invention a pour objet une utilisation cosmétique ou encore non thérapeutique, d'une quantité efficace :
- d'au moins un polypeptide choisi parmi les fragments de SEQ ID N°2 de longueur d'au moins 5 acides aminés consécutifs, et les analogues d'un des fragments, lesdits fragments et analogues présentant au moins un domaine glutamine (Gln) - lysine (Lys),
à titre d'agent utile pour renforcer la fonction barrière de l'épiderme et/ou prévenir et/ou traiter les signes de la sècheresse cutanée et/ou encore prévenir et/ou traiter les signes du vieillissement cutané.

Selon un autre aspect, la présente invention a également pour objet une utilisation :
- d'au moins un polypeptide choisi parmi les fragments de SEQ ID N°2 de longueur d'au moins 5 acides aminés consécutifs, et les analogues d'un des fragments, lesdits fragments et analogues présentant au moins un domaine glutamine (Gln) - lysine (Lys),
pour fabriquer une composition thérapeutique destinée à:
- renforcer la fonction barrière de l'épiderme et prévenir et/ou traiter les signes de la sècheresse cutanée ou encore prévenir et/ou traiter les signes du vieillissement cutané ; et/ou
- prévenir et/ou traiter les troubles trophiques cutanés ou ceux consécutifs à des troubles de la cicatrisation; et/ou
- prévenir et/ou traiter l'amincissement de l'épiderme et en particulier de la couche cornée et/ou à traiter une fragilité excessive du revêtement cutané et/ou à induire l'épaississement de la couche cornée ; et/ou
- traiter l'hyperkératose, la xérose, les ichtyoses, le psoriasis, les lésions tumorales hyperkératosiques bénignes ou malignes ou les kératoses réactionnelles.

La présente invention a également pour objet l'un des polypeptides décrits ci-dessus pour son utilisation pour prévenir et/ou traiter les désordres mentionnés ci-avant.

Est également décrite une composition cosmétique ou pharmaceutique comprenant, dans un milieu physiologiquement acceptable, au moins un polypeptide isolé naturel ou synthétique, ledit polypeptide comprenant au moins un fragment de SEQ ID N°2 de longueur d'au moins 5 acides aminés consécutifs, ou un analogue d'un des fragments, lesdits fragments et analogues présentant au moins un domaine glutamine (Gln) - lysine (Lys).

Est également décrit un polynucléotide isolé comprenant au moins un fragment de SEQ ID N°1, ou un analogue d'un des fragments.

Par renforcer la fonction barrière cutanée, on entend assurer le maintien de la fonction barrière de la peau à un niveau minimum d'efficacité correspondant à son niveau d'efficacité normale, c'est-à-dire le niveau auquel elle assure sa fonction de protection de l'organisme.

Par « signes de sècheresse cutanée », on entend toutes les modifications de l'aspect extérieur de la peau dues notamment à la déshydratation de l'épiderme, telles que l'aspect terne, rugueux, non soyeux, rougeâtre et/ou écailleux, ainsi que la perte de souplesse et une diminution de l'épaisseur de la peau. Dans des cas sévères, les signes de sécheresse cutanée incluent les sensations liées au phénomène de sécheresse, telles que les démangeaisons, les picotements et/ou les tiraillements, pouvant conduire à l'apparition de réelles pathologies telles que, par exemple, l'hypersensibilité, l'atrophie cutanée, les dermites atopiques ou les xéroses hivernales.

Par « prévenir et/ou traiter les signes de la sècheresse cutanée », on entend par conséquent renforcer, préserver et/ou restaurer la barrière épidermique, notamment sa fonction protectrice ou régulatrice, améliorer et/ou de renforcer la cohésion cellulaire de l'épiderme, augmenter la résistance épidermique aux agents agresseurs, améliorer et/ou renforcer la structure de l'épiderme, augmenter son épaisseur, et/ou garantir l'intégrité cutanée.

Par « prévenir et/ou traiter les signes du vieillissement cutané », on entend de façon préférentielle le vieillissement photo-induit ou photo-vieillissement. Par « signes du vieillissement cutané », on entend toutes les modifications de l'aspect extérieur de la peau dues au vieillissement comme, par exemple, les rides et les ridules, la peau flétrie, la peau molle, la peau amincie, le manque d'élasticité et/ou de fermeté de la peau, la peau terne et sans éclat.

Par « quantité efficace » au sens de la présente invention, on entend la quantité minimale nécessaire à l'observation de l'effet attendu, à savoir un effet cosmétique ou un effet thérapeutique, étant entendu que les quantités efficaces nécessaires à l'obtention d'un effet cosmétique ou d'un effet thérapeutique peuvent être, le cas échant, identiques ou différentes.
Par « troubles trophiques cutanés », on entend les troubles notamment d'origine vasculaires, les anomalies de la peau (épiderme, derme et hypoderme) ou lésions engendrées par une insuffisance microcirculatoire d'origine artérielle ou veineuse. On les appelle troubles trophiques car ils résultent d'une mauvaise nutrition de la peau qui est mal irriguée.

### Polypeptides selon l'invention

Par « polypeptide », on entend une molécule comprenant une chaîne linéaire d'acides aminés liés les uns aux autres par des liaisons peptidiques.

Dans la présente description, on utilisera le terme polypeptide pour désigner également une protéine ou un peptide.

Les polypeptides selon l'invention ont une longueur inférieure ou égale à 80 acides aminés. De préférence, le polypeptide consiste en 4 à 20, plus préférentiellement 7 à 15 acides aminés.

Une des caractéristiques essentielles de ce polypeptide est que celui-ci doit présenter au moins un domaine glutamine (Q, Gln) - lysine (K, Lys) afin de pouvoir servir de substrat aux transglutaminases.

Selon l'invention, le polypeptide est choisi parmi les fragments de SEQ ID N°2 de longueur d'au moins 5 acides aminés consécutifs et les analogues des fragments, lesdits fragments et analogues présentant au moins un domaine glutamine (Gln) - lysine (Lys).

Au sens de la présente invention, on entend désigner de manière générale, sauf indication contraire, par LCE6A la séquence SEQ ID N°2 de la protéine ayant ou non subi des modifications post-traductionnelles.

Le polypeptide selon l'invention est un polypeptide isolé. Par polypeptide « isolé » on entend un polypeptide isolé du corps humain ou d'un organisme vivant, de préférence sous forme purifiée.
Par « analogue d'un polypeptide », on entend désigner tout polypeptide présentant (a) une homologie de séquence d'acides aminés, en particulier vis-à-vis d'une des séquences caractéristiques dudit polypeptide ou de ses fragments, ainsi que (b) une activité biologique de même nature. L'homologie est d'au moins 80 %, et peut être par exemple d'au moins 85 %, et par exemple d'au moins 95 %. Cette homologie de séquence peut résulter de modifications issues d'une ou plusieurs mutations (substitutions, insertions ou délétions) dans les séquences des polypeptides selon l'invention. De préférence, lesdits polypeptides comprennent seulement des mutations du type substitution. Ces substitutions peuvent être conservatrices ou non. Par pourcentage d'homologie, on entend le nombre de résidus identiques entre deux séquences, lesdites séquences étant alignées de façon à obtenir une correspondance maximale. Différents algorithmes connus de l'art antérieur peuvent être utilisés pour mesurer l'homologie entre deux séquences. Par exemple, les séquences de polynucléotides peuvent être comparées en utilisant FASTA, Gap ou Bestfit, qui sont des programmes du Wisconsin Package Version 10.0, Genetics Computer Group (GCG), Madison, Wis. Alternativement, les séquences peuvent être comparées en utilisant le programme BLAST (Altschul et al., J. Mol. Biol. 215:403-410 (1990); Gish and States, Nature Genet. 3:266-272 (1993); Madden et al., Meth. Enzymol. 266:131-141 (1996); Altschul et al., Nucleic Acids Res. 25:3389-3402 (1997); Zhang and Madden, Genome Res. 7:649-656 (1997)), particulièrement blastp ou tblastn (Altschul et al., Nucleic Acids Res. 25:3389-3402 (1997)).

Par « activité biologique de même nature », on entend désigner en particulier un analogue ou un fragment de séquence d'acides aminés de polypeptide selon l'invention présentant au moins une des caractéristiques ou propriétés fonctionnelles des polypeptides selon l'invention, notamment en ce que : (i) il est capable d'être reconnu par un anticorps spécifique d'un polypeptide selon l'invention; (ii) il présente au moins l'un des domaines ou régions tels que définis ci-après ; (iii) il est capable de lier les transglutaminases (TGM) telles que les TGM 1, 3 et 5 humaines qui participent à la formation de l'enveloppe cornée.

Selon un mode de réalisation préféré, l'analogue du polypeptide selon l'invention diffère de la séquence de la protéine LCE6A (SEQ ID N°2) uniquement par la présence de substitutions conservatrices. A titre d'exemple de mutations conservatrices que l'on peut considérer dans la présente invention, il peut être mentionné, de manière non exhaustive, le remplacement d'un ou plusieurs résidus d'acides aminés par un acides aminés de même classe, telles que des substitutions d'acides aminés aux chaînes latérales non chargées (tel que l'asparagine, la glutamine, la serine, la cystéine et la tyrosine), d'acides aminés aux chaînes latérales basiques (tels que la lysine, l'arginine, et l'histidine), d'acides aminés aux chaînes latérales acides (tels que l'acide aspartique et l'acide glutamique), d'acides aminés aux chaînes latérales apolaires (tels que l'alanine, la valine, la leucine, l'isoleucine, la proline, la phénylalanine, la méthionine et le tryptophane).

Un polypeptide ou analogue également visé par la présente invention peut être un polypeptide ayant subi une ou plusieurs modification(s) post-traductionnelle(s).

Par " modification(s) post-traductionnelle(s) ", on entend englober l'ensemble des modifications qu'un polypeptide est susceptible de subir à l'issue de sa synthèse dans une cellule, telles que, par exemple, une ou des phosphorylation(s), une ou des thiolation(s), une ou des acétylation(s), une ou des glycosylation(s), une ou des lipidation(s), telles qu'une palmitoylation, un réarrangement structural de type formation de ponts disulfures à l'intérieur de la séquence peptidique.

Au sens de l'invention, on entend désigner par "fragment de polypeptide" toute portion d'un polypeptide conforme à l'invention comprenant au moins 5, de préférence au moins 7, de préférence au moins 9, et plus particulièrement au moins 15 acides aminés consécutifs dudit polypeptide, ladite portion présentant en outre une activité biologique de même nature.

Selon un mode de réalisation préférentiel, un fragment de polypeptide convenant à l'invention est choisi parmi les séquences SEQ ID N°3, SEQ ID N°4, SEQ ID N°5, et leurs mélanges.

Selon un autre mode de réalisation, un polypeptide convenant à la mise de l'invention est également un polypeptide tel que défini précédemment, fusionné avec un autre polypeptide, un agent de ciblage hydrophile ou hydrophobe, un précurseur de bioconversion, un agent de marquage luminescent, radioactif ou colorimétrique. De manière non limitative, on peut citer comme exemple de composés susceptibles d'être couplés avec un polypeptide conforme à l'invention des protéines fluorescentes, des composés chimiques fluorescents tels que la rhodamine, la fluorescéine, des composés phosphorescents, des éléments radioactifs ou des agents de marquage colorimétrique comme les substrats chromogènes sensibles à l'action de certaines enzymes telles que la galactosidase, la peroxydase, l'acétyltransférase.

Selon la nature des composés susceptibles d'être couplés avec un polypeptide conforme à l'invention, le couplage peut être réalisé par des procédés chimiques, notamment au moyen de fonctions chimiques réactives ou par des procédés de biologie moléculaire connus de l'homme de l'art.

Le polypeptide selon l'invention peut en outre comporter une ou plusieurs modification(s) chimique(s) améliorant sa résistance à la dégradation ou sa biodisponibilité. Cette modification doit être biologiquement compatible et doit être compatible avec une utilisation dans le domaine des cosmétiques ou de la pharmacie. De telles modifications chimiques ou enzymatiques sont bien connues de l'homme du métier. De manière non limitative, on peut citer par exemple : les modifications de l'extrémité C- ou N-terminales des polypeptides (acétylation); les modifications de la liaison entre deux acides aminés (acylation ou alkylation) ; des changements de chiralité. De préférence, on utilise une protection basée soit sur l'acylation ou l'acétylation de l'extrémité amino-terminale, soit sur l'amidation ou l'estérification de l'extrémité carboxy-terminale, soit encore des deux.

Ainsi, l'invention concerne un polypeptide tel que défini précédemment, ledit polypeptide étant sous forme protégée ou non.

Dans le domaine des acides aminés, la géométrie des molécules est telle qu'elles peuvent théoriquement se présenter sous la forme d'isomères optiques différents. Il existe, en effet, une conformation moléculaire de l'acide aminé (AA) telle qu'elle dévie à droite le plan de polarisation de la lumière (conformation dextrogyre ou D-aa), et une conformation moléculaire de l'acide aminé (aa) telle qu'elle dévie à gauche le plan de polarisation de la lumière (conformation lévogyre ou L-aa). La nature n'a retenu pour les acides aminés naturels que la conformation lévogyre. En conséquence, un polypeptide d'origine naturelle ne sera constitué que d'acides aminés de type L-aa. Cependant la synthèse chimique en laboratoire permet de préparer des acides aminés ayant les deux conformations possibles. A partir de ce matériel de base, il est ainsi possible d'incorporer lors de la synthèse de polypeptide aussi bien des acides aminés sous forme d'isomères optiques dextrogyre ou lévogyre.

Ainsi, les acides aminés constituant le polypeptide selon l'invention peuvent être sous configuration L- et D- ; de manière préférentielle, les acides aminés sont sous forme L. Le polypeptide selon l'invention peut donc être sous forme L-, D- ou DL-.

Les polypeptides selon l'invention peuvent être d'origine naturelle ou synthétique. Ils peuvent être synthétisés par n'importe quelle méthode bien connue de l'homme du métier. De telles méthodes incluent notamment la synthèse chimique classique (en phase solide ou en phase homogène liquide), la synthèse enzymatique (Kullman et ai, J. Biol. Chem. 1980, 225, 8234) à partir d'acides aminés constitutifs ou de leurs dérivés.

Les polypeptides selon l'invention peuvent également être obtenus par des procédés de production biologique tels que la fermentation d'une souche de bactéries modifiées ou non, par génie génétique pour produire les polypeptides ou fragments selon l'invention à l'aide de techniques recombinantes (cf. exemples), ou encore par extraction de protéines d'origine animale ou végétale suivie d'une hydrolyse contrôlée qui libère les fragments peptidiques de tailles moyennes et de petites tailles, objet de l'invention. D'autres procédés plus simples ou plus complexes peuvent être envisagés par l'homme du métier connaissant le métier de synthèse d'extraction et de purification des protéines et des peptides.

De préférence, les polypeptides selon l'invention sont obtenus par une synthèse chimique, cette technologie étant particulièrement avantageuse pour des raisons de pureté, de spécificité antigénique, d'absence de produits de réaction secondaires non désirés et pour sa facilité de production.

Selon un mode de réalisation avantageux de l'invention, les polypeptides précités selon l'invention sont préalablement solubilisés dans un ou plusieurs solvants cosmétiquement ou pharmaceutiquement acceptables. De tels solvants sont classiquement utilisés par l'homme du métier, comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

Selon un autre mode de réalisation avantageux de l'invention, les polypeptides précités sont préalablement solubilisés dans un vecteur cosmétique ou pharmaceutique comme les liposomes ou adsorbés sur des polymères organiques poudreux, des supports minéraux comme les talcs et bentonites, et plus généralement solubilisés dans, ou fixés sur, tout vecteur cosmétiquement ou pharmaceutiquement acceptable.

### La séquence d'acides nucléiques selon l'invention

Selon un mode de réalisation, la présente invention concerne également des séquences d'acides nucléiques codant pour un polypeptide de l'invention et leur mise en oeuvre dans les différentes utilisations conformes à l'invention.

Le terme « acide nucléique » signifie un enchaînement (brins) d'au moins deux désoxyribo-nucléotides ou ribonucléotides comprenant éventuellement au moins un nucléotide modifié permettant l'hybridation, comprenant par exemple une liaison modifiée, une base purique ou pyrimidique modifiée, ou un sucre modifié.

L'acide nucléique peut être de l'acide désoxyribonucléique (ADN) ou de l'acide ribonucléique (ARN), ou un mélange des deux. Il peut être sous forme simple chaîne (simple brin) ou en duplexe (double brins), ou un mélange des deux.

Ainsi, il est décrit l'utilisation de séquences d'acides nucléiques codant pour un polypeptide conforme à l'invention, notamment les séquences correspondant au moins à un fragment de la séquence d'acides nucléiques représentée par SEQ ID N°1, ou des analogues de celle-ci pour la préparation d'une composition conforme à l'invention.

Au sens de la présente invention, on entend désigner par « fragment de séquence d'acides nucléiques », une séquence d'acides nucléiques codant pour une partie d'un polypeptide conforme à l'invention, ou un analogue de celle-ci.

Par « analogue d'une séquence d'acides nucléiques », on entend désigner toute séquence d'acides nucléiques, éventuellement résultant de la dégénérescence du code des acides nucléiques, et codant pour un polypeptide de séquence identique ou analogue à celle du polypeptide codée par ladite séquence d'acides nucléiques.

L'acide nucléique peut être naturel ou synthétique, un oligonucléotide, un polynucléotide, un fragment d'acide nucléique, un ARN messager, un acide nucléique obtenu par une technique d'amplification enzymatique telle que la PCR (Polymerase Chain Reaction).

Les séquences d'acides nucléiques peuvent être issues de toutes origines possibles, à savoir soit animale, en particulier de mammifères et encore plus particulièrement humaine, soit végétale, soit de micro-organismes (virus, phages, bactéries entre autres) ou encore de champignons, sans préjuger du fait qu'elles soient présentes de manière naturelle ou non dans ledit organisme d'origine.

Selon un mode de réalisation préféré, les polynucléotides selon l'invention peuvent être utilisés comme amorce et/ou sonde dans des procédés mettant en oeuvre notamment la technique PCR.

Cette technique nécessite le choix de paires d'amorces oligonucléotidiques encadrant le fragment qui doit être amplifié. Les fragments amplifiés peuvent être identifiés, par exemple après une électrophorèse en gel d'agarose ou de polyacrylamide, ou après une technique chromatographique comme la filtration sur gel ou la chromatographie échangeuse d'ions. La spécificité de l'amplification peut être contrôlée par hybridation moléculaire en utilisant comme sonde les séquences nucléotidiques de polynucléotides de l'invention, des plasmides contenant ces séquences ou leurs produits d'amplification.

Les fragments nucléotidiques amplifiés peuvent être utilisés comme réactifs dans des réactions d'hybridation afin de mettre en évidence la présence, dans un échantillon biologique, d'un acide nucléique cible de séquence complémentaire à celle desdits fragments nucléotidiques amplifiés.

L'invention vise également les fragments nucléotidiques susceptibles d'être obtenus par amplification à l'aide d'amorces selon l'invention.

D'autres techniques d'amplification de l'acide nucléique cible peuvent être avantageusement employées comme alternative à la PCR (PCR-like) à l'aide de couple d'amorces de séquences nucléotidiques selon l'invention. Par PCR-like on entendra désigner toutes les méthodes mettant en oeuvre des reproductions directes ou indirectes des séquences d'acides nucléiques, ou bien dans lesquelles les systèmes de marquage ont été amplifiés, ces techniques sont bien entendu connues, en général il s'agit de l'amplification de l'ADN par une polymérase ; l'échantillon d'origine est un ARN il convient préalablement d'effectuer une transcription inverse.

Dans le cas où le polynucléotide cible à détecter est un ARN, par exemple un ARNm, on utilisera avantageusement, préalablement à la mise en oeuvre d'une réaction d'amplification à l'aide des amorces selon l'invention ou à la mise en oeuvre d'un procédé de détection à l'aide des sondes de l'invention, une enzyme de type transcriptase inverse afin d'obtenir un ADN complémentaire (ADNc) à partir de l'ARN contenu dans l'échantillon biologique. L'ADNc obtenu servira alors de cible pour les amorces ou les sondes mises en oeuvre dans le procédé d'amplification ou de détection selon l'invention.

En l'occurrence, l'invention se rapporte également à l'utilisation de fragments d'acides nucléiques isolés et purifiés codant pour les polypeptides considérés selon l'invention.

Une séquence d'acides nucléiques conforme à l'invention peut comprendre une séquence sens, anti-sens ou interférentielle correspondant à une séquence codant pour un polypeptide conforme à l'invention.

Ainsi, il est décrit l'utilisation de séquences d'acides nucléiques, notamment d'acides désoxyribonucléiques, ou d'acides ribonucléiques, codant pour un polypeptide conforme à l'invention. Les séquences d'acides nucléiques peuvent notamment être utilisées pour préparer les séquences d'acides ribonucléiques correspondantes, sens ou anti-sens.

La quantité de polypeptide ou de séquence d'acides nucléiques conformes à l'invention contenue dans une composition selon l'invention, encore dite « quantité efficace » est, bien entendu, fonction de la nature du composé et de l'effet recherché et peut donc varier dans une large mesure. Pour donner un ordre de grandeur, une composition peut contenir un polypeptide ou une séquence d'acides nucléiques conforme à l'invention en une quantité représentant de 0,00001 % à 20 % du poids total de la composition, en particulier en une quantité représentant de 0,0001 % à 5 % du poids total de la composition, et plus particulièrement en une quantité représentant de 0,003 % à 3 % du poids total de la composition.

### La composition selon l'invention

De façon préférée, la composition selon l'invention est appliquée sur une peau sèche pathologique ou non pathologique. Elle peut être avantageusement appliquée sur la peau du visage, du cou et éventuellement du décolleté ou en variante sur une partie quelconque du corps.

La composition cosmétique et/ou pharmaceutique peut être appliquée le matin et/ou le soir, sur l'ensemble du visage, du cou et éventuellement du décolleté voire du corps.

La composition cosmétique et/ou pharmaceutique mise en oeuvre selon l'invention comprend généralement un milieu physiologiquement acceptable et de préférence cosmétiquement acceptable, c'est-à-dire qui convient à une utilisation en contact avec la peau humaine sans risque de toxicité, d'incompatibilité, d'instabilité, de réponse allergique et notamment qui ne provoque pas de sensations d'inconfort (rougeurs, tiraillements, picotements...) inacceptables pour l'utilisateur.

De préférence, le milieu physiologiquement acceptable est constitué d'eau.

La composition cosmétique et/ou pharmaceutique utilisée selon l'invention peut se présenter sous toute forme adaptée à une application topique sur la peau et en particulier sous forme d'émulsion huile-dans-eau, eau-dans-huile ou multiple (E/H/E ou H/E/H), qui peuvent éventuellement être des microémulsions ou des nanoémulsions, ou sous forme d'hydrodispersion, de solution, de gel aqueux ou de poudre. De préférence, cette composition se présente sous la forme d'une émulsion huile-dans-eau.

Cette composition lorsqu'elle est utilisée en tant que produit de soin ou de nettoyage de la peau du visage et/ou du corps et elle peut se présenter notamment sous forme de fluide, de gel ou de mousse, conditionnés par exemple dans un flacon-pompe, un aérosol ou un tube, ou de crème conditionnée par exemple dans un pot. En variante, elle peut avoir la forme d'un produit de maquillage et en particulier d'un fond de teint ou d'une poudre libre ou compacte.

Outre le polypeptide précédemment décrit, la composition cosmétique et/ou pharmaceutique selon l'invention peut également comprendre au moins un additif usuel dans le domaine cosmétique ou de la pharmacie, tel que par exemple un composé choisi parmi un agent gélifiant et/ou épaississant, un agent tensioactif ou co-tensioactif, un corps gras liquide ou une huile, une cire, un élastomère de silicone, un filtre solaire, un colorant, un agent matifiant ou une charge, un pigment, un agent tenseur, un conservateur, un séquestrant, un parfum et leurs mélanges.

Notamment, selon un mode de réalisation préférée, la composition cosmétique selon l'invention peut comprendre, de manière non limitative, un ou plusieurs des additifs suivants :
- un ou plusieurs agent(s) gélifiant(s) et/ou épaississant(s) de la phase aqueuse, choisi par exemple parmi les homo- et copolymères réticulés ou non, hydrophiles ou amphiphiles, d'acide acryloylméthylpropane sulfonique (AMPS) et/ou d'acrylamide et/ou d'acide acrylique et/ou de sels ou d'esters d'acide acrylique tels que les ammonium acryloyldiméthyltaurate/VP copolymer et ammonium acryloyldiméthyl-taurate/beheneth-25 méthacrylate copolymer, notamment ceux vendus sous les dénominations Aristoflex^{®} AVC et HMB de Clariant, ou encore les acrylates/C10-30 Alkyl Acrylate Crosspolymer vendu sous la dénomination commerciale PEMULEN^{®} TR-1 ou TR-2, Carbopol^{®} 1382, Carbopol^{®} Ultrez 20 par la société Novéon, les dérivés cellulosiques, les gommes d'origine végétale (acacia ou arabique, agar, guar, caroube, alginates, carraghénanes, pectine) ou d'origine microbienne (xanthane, pullulane), les argiles (laponite). Ledit agent gélifiant et/ou épaississant peut être présent dans la composition en une teneur de l'ordre de 0,01 à 5% en poids, par rapport au poids total de la composition ;
- un ou plusieurs agent(s) tensioactif(s), de préférence émulsionnants, qu'ils soient non ioniques, anioniques, cationiques ou amphotères, et en particulier les esters d'acides gras et de polyols tel que les esters d'acides gras et de glycérol oxyalkylénés (plus particulièrement polyoxyéthylénés), les esters d'acides gras et de sorbitan oxyalkylénés, les esters d'acides gras oxyalkylénés (oxyéthylénés et/ou oxypropylénés) comme le mélange PEG-100 Stearate/Glyceryl Stearate commercialisé par exemple par la société Croda sous la dénomination Arlacel^{®} 165 et les esters d'acides gras et de sucrose comme le stéarate de sucrose; les éthers d'alcool gras et de sucre, notamment les alkylpolyglucosides (APG) tels que le décylglucoside et le laurylglucoside, le cétostéarylglucoside éventuellement en mélange avec l'alcool cétostéarylique, commercialisé par exemple sous la dénomination Montanov^{®} 68 par la société Seppic, ainsi que l'arachidyl glucoside, par exemple sous la forme du mélange d'alcools arachidique et béhénique et d'arachidylglucoside commercialisé sous la dénomination Montanov^{®} 202 par la société Seppic ; les éthers d'alcools gras et de polyéthylèneglycol; les polysiloxanes modifiés polyéthers; la bétaïne et ses dérivés; les polyquaterniums; les sels de sulfate d'alcools gras éthoxylés; les sulfosuccinates; les sarcosinates; les alkyl- et dialkylphosphates et leurs sels; et les savons d'acides gras. Ledit agent tensioactif peut être présent dans la composition dans une teneur de l'ordre de 0,1 à 8%, de préférence 0,5 à 3% en poids, par rapport au poids total de la composition ;
- un ou plusieurs co-tensioactif(s) tels que les alcools gras linéaires à longue chaîne carbonée (C14-C20) et en particulier les alcools cétylique et stéarylique, ledit agent tensioactif étant présent dans la composition à raison de 0,1 à 5%, de préférence 0,5 à 2% en poids, par rapport au poids total de la composition ;
- un ou plusieurs corps gras liquide(s) à température ambiante, communément dénommé(s) huiles(s), volatile(s) ou non volatile(s), hydrocarboné(s), siliconée(s), linéaire(s), cyclique(s) ou ramifiée(s), par exemple, les huiles de silicone telles que les polydiméthylsiloxanes (diméthicones), les polyalkylcyclosiloxanes (cyclométhicones) et les polyalkylphénylsiloxanes (phenyldiméthicones); les huiles synthétiques telles que les huiles fluorées, les alkyl benzoates et les hydrocarbures ramifiés tels que le polyisobutylène, l'isododécane; les huiles minérales (paraffine); végétales (huile d'amande douce, de macadamia, de pépin de cassis, de jojoba ou encore de camelina sativa telle que l'huile vendue sous la dénomination commerciale Lipex^{®} Omega 3/6 par la société Unipex) ; les alcools gras, les amides grasses, les acides ou les esters gras comme le benzoate d'alcools en C12-C15 vendu sous la dénomination commerciale Finsolv^{®} TN par la société Innospec, les triglycérides dont ceux des acides caprique/caprylique, le dicaprylyl carbonate vendu sous la dénomination Cetiol^{®} CC par la société Cognis ; de préférence à raison de 0,1 à environ 10%, de préférence, de 0,5 à 5% en poids, par rapport au poids total de la composition ;
- une ou plusieurs cires (composé solide ou substantiellement solide à température ambiante), et dont le point de fusion est généralement supérieur à 35°C, telles que l'ozokérite, la cire de polyéthylène, la cire d'abeille ou la cire de carnauba, de préférence à raison de 0,01 à environ 5%, de préférence 0,5 à 5% en poids, par rapport au poids total de la composition ;

- un ou plusieurs élastomère(s) de silicone obtenus notamment par réaction, en présence d'un catalyseur, d'un polysiloxane ayant au moins un groupe réactif (hydrogène ou vinyle, notamment) et portant au moins un groupe alkyle (notamment méthyle) ou phényle, terminal et/ou latéral, avec un organosilicone tel qu'un organohydrogénopolysiloxane, de préférence à raison de 0,1 à environ 20%, de préférence 0,25 à 15% en poids, par rapport au poids total de la composition ;
- un ou plusieurs filtre(s) solaire(s), notamment les filtres organiques, tels que les dérivés de dibenzoylméthane (dont le butyl méthoxydibenzoylméthane vendu en particulier par DSM sous le nom commercial Parsol^{®} 1789), les dérivés d'acide cinnamique (dont l'éthylhexyl méthoxycinnamate vendu en particulier par DSM sous le nom commercial Parsol^{®} MCX), les salicylates, les acides para-aminobenzoïques, les β-β' - diphénylacrylates, les benzophénones, les dérivés de benzylidène camphre, les phénylbenzimidazoles, les triazines, les phénylbenzotriazoles et les dérivés anthraniliques; ou les filtres inorganiques, à base d'oxydes minéraux sous forme de pigments ou de nanopigments, enrobés ou non, et en particulier à base de dioxyde de titane ou d'oxyde de zinc; de préférence à raison de 0,1 à environ 30%, mieux, de 0,5 à 20% en poids, par rapport au poids total de la composition ;
- un ou plusieurs colorant(s) hydrosoluble(s) tels que, par exemple, le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine ou la xanthophylle, de préférence à raison de 0,1 à environ 2% en poids, par rapport au poids total de la composition ;
- une ou plusieurs charges, en particulier des agents matifiants ou des charges à effet flouteur, et en particulier les poudres à effet soft-focus.

Par charge, il faut comprendre des particules incolores ou blanches, minérales ou de synthèse, lamellaires ou non lamellaires, adaptées à donner du corps ou de la rigidité à la composition et/ou de la douceur, de la matité et de l'uniformité immédiate à l'application. Ces charges peuvent notamment modifier voire masquer les rides par un effet de camouflage, ou un effet de floutage.

Les agents matifiants peuvent être choisis parmi les polymères matifiants (en solution, en dispersion ou sous forme de particules) et les particules inorganiques qui réduisent la brillance de la peau et unifient le teint. L'agent matifiant pourra notamment être choisi parmi un amidon, le talc, des microbilles de cellulose, des fibres végétales, des fibres synthétiques, en particulier de polyamides (les poudres de Nylon^{®} telles que Nylon-12 (Orgasol^{®} commercialisé par la société Atochem), des microsphères de copolymères acryliques notamment de poly(méth)acrylate de méthyle (particules PMMA ou les particules Micropearl^{®} M310 vendue par la société Seppic), les poudres de silice, les poudres de résine de silicone, les poudres de polymères acryliques, les poudres de polyéthylène, les organopolysiloxanes réticulés élastomères (commercialisés notamment sous les dénominations KSG^{®} par la société Shin-Etsu, sous les dénominations Trefil^{®}, BY29^{®} ou EPSX^{®} par la société Dow Corning ou sous les dénominations Gransil^{®} par la société Grant Industries), les poudres composites de talc/dioxyde de titane/alumine/silice, les poudres de silicates, et leurs mélanges.

La charge à effet « soft focus » peut donner de la transparence au teint et un effet flou. De préférence, les charges « soft focus » ont une taille moyenne de particules inférieure ou égale à 30 microns, plus préférentiellement inférieure ou égale à 15 microns. Ces charges « soft focus » peuvent être de toutes formes et en particulier être sphériques ou non sphériques. Elles peuvent être choisies parmi les poudres de silice et silicates, notamment d'alumine, les poudres de type polyméthyl méthacrylate (PMMA ou Micropearl^{®} M310), le talc, les composites silice/TiO2 ou silice/oxyde de zinc, les poudres de polyéthylène, les poudres d'amidon, les poudres de polyamides, les poudres de copolymères styrène/acrylique, les élastomères de silicone, et leurs mélanges.

De préférence ces agents matifiants ou charges à effet soft-focus sont utilisés à raison de 0,1 à environ 10% en poids, par rapport au poids total de la composition, de préférence à raison de 0,1 à environ 7% en poids.

Une ou plusieurs pigments blancs ou colorés, nacrés ou non, minéraux et/ou organiques, enrobés ou non, insolubles dans le milieu, destinées à colorer et/ou opacifier la composition. Ils peuvent être de taille usuelle ou nanométrique. On peut citer, parmi les pigments minéraux, le dioxyde de titane, éventuellement traité en surface, les oxydes de fer ou de chrome, le violet de manganèse, le bleu outremer, l'hydrate de chrome et le bleu ferrique. Parmi les pigments organiques, on peut citer le noir de carbone, les pigments de type D&C, et les laques à base de carmin de cochenille, de baryum, strontium, calcium, aluminium. Les pigments nacrés ou nacres sont des particules irisées qui réfléchissent la lumière. Ces pigments nacrés peuvent être choisis parmi les pigments nacrés blancs tels que le mica recouvert de titane, ou d'oxychlorure de bismuth, les pigments nacrés colorés tels que le mica titane avec des oxydes de fer. Les pigments peuvent avoir subi un traitement de surface. De préférence, ces pigments sont utilisés à raison de 0,1 à environ 10% en poids, par rapport au poids total de la composition, de préférence à raison de 0,1 à environ 5% en poids.
- un ou plusieurs agents tenseurs. Par agent tenseur, il faut comprendre un composé adapté à tendre la peau et, par cet effet de tension, lisser la peau et y faire diminuer voire disparaître de façon immédiate les rides et les ridules. Comme agents tenseurs, on peut citer les polymères d'origine naturelle; les silicates mixtes ; les particules colloïdales de charges inorganiques ; les polymères synthétiques ; et leurs mélanges. On peut citer notamment : les polymères d'origine végétale ou microbienne, les polymères issus des phanères, les protéines d'oeuf et les latex d'origine naturelle. Ces polymères sont de préférence hydrophiles. Comme polymères d'origine végétale, on peut citer en particulier les protéines et hydrolysats de protéines, et plus particulièrement les extraits de céréales, de légumineuses et d'oléagineuses, tels que les extraits de maïs, de seigle, de froment, de sarrasin, de sésame, d'épeautre, de pois, de tapioca, de fève, de lentille, de soja et de lupin. D'autres agents tenseurs pouvant être mis en oeuvre selon l'invention sont les polysaccharides d'origine naturelle, notamment l'amidon issu notamment de riz, de maïs, de tapioca, de pomme de terre, de manioc, de pois ; les carraghénanes, gommes d'acacia (gomme arabique), alginates, agars, gellanes, gommes xanthanes, polymères cellulosiques et pectines, avantageusement en dispersion aqueuse de microparticules de gel, les dérivés cellulosiques, et leurs mélanges. Les polymères synthétiques se présentent généralement sous forme d'un latex ou d'un pseudolatex et peuvent être de type polycondensat ou obtenus par polymérisation radicalaire. On peut citer notamment les dispersions de polyester/polyuréthane et de polyéther/polyuréthane. De préférence, l'agent tenseur est un copolymère de PVP/dimethiconylacrylate et de polyuréthane hydrophile (Aquamere^{®} S-2011^{®} de la société Hydromer).
- un ou plusieurs conservateur(s) ;
- les séquestrants tels que les sels d'EDTA;
- les parfums;
- et leurs mélanges.

Des exemples de tels additifs sont cités notamment dans le Dictionnaire CTFA (International Cosmetic Ingredient Dictionary and Handbook publié par The Cosmetic, Toiletry and Fragrance Association, 11ème Edition, 2006) qui décrit une grande variété, sans limitation, d'ingrédients cosmétiques et pharmaceutiques habituellement utilisés dans l'industrie du soin de la peau, qui conviennent pour être utilisés comme ingrédients additionnels dans les compositions selon la présente invention.

L'homme du métier est en mesure de choisir, parmi l'ensemble de ces éventuels additifs, aussi bien la composition que la quantité de ceux qui seront ajoutés à la composition, de telle sorte que celle-ci conserve l'ensemble de ses propriétés.

En outre, la composition selon la présente invention peut éventuellement contenir divers agents actifs qui peuvent être choisis dans le groupe constitué des vitamines, des antioxydants, des agents hydratants, des agents anti-pollution, des agents kératolytiques, des astringents, des anti-inflammatoires, des agents blanchissants et des agents favorisant la microcirculation.

Des exemples de vitamines incluent les vitamines A, B1, B2, B6, C et E et leurs dérivés, l'acide pantothénique et ses dérivés et la biotine.

Des exemples d'antioxydants incluent l'acide ascorbique et ses dérivés tels que le palmitate d'ascorbyle, le tétraisopalmitate d'ascorbyle, l'ascorbyl glucoside, le magnésium ascorbyl phosphate, le sodium ascorbyl phosphate et le sorbate d'ascorbyle; le tocophérol et ses dérivés, tels que l'acétate de tocophérol, le sorbate de tocophérol et d'autres esters de tocophérol; le BHT et BHA; les esters de l'acide gallique, l'acide phosphorique, l'acide citrique, l'acide maléique, l'acide malonique, l'acide succinique, l'acide fumarique, la céphaline, l'hexamétaphosphate, l'acide phytique, et les extraits de plantes, par exemple de racines de Zingiber Officinale (Gingembre) tel que le Blue Malagasy Ginger commercialisé par la société BIOLANDES, de Chondrus crispus, Rhodiola, Thermus thermophilus, la feuille de maté, le bois de chêne, l'écorce de Rapet Kayu, les feuilles de Sakura et les feuilles d'ylang ylang.

Des exemples d'agents hydratants incluent le polyéthylène glycol, le propylène glycol, le dipropylène glycol, la glycérine, le butylène glycol, le xylitol, le sorbitol, le maltitol, les mucopolysaccharides, tels que l'acide chondroïtine sulfurique, l'acide hyaluronique de haut ou de bas poids moléculaire ou encore l'acide hyaluronique potentialisé par un dérivé de silanol tel que l'actif Epidermosil^{®} commercialisé par la société Exymol, et l'acide mucoitinsulfurique; l'acide caronique; les sels biliaires, une composante principale du FHN (facteur d'hydratation naturelle) comme un sel de l'acide pyrrolidone carboxylique et un sel d'acide lactique, un analogue d'acide aminé tel que l'urée, la cystéine et la sérine; un collagène soluble à chaîne courte, les PPG diglycérine, les homo- et copolymères de 2-méthacryloyloxyéthylphosphorylcholine comme le Lipidure HM et le Lipidure PBM de NOF; l'allantoïne; des dérivés de glycérine tels que le PEG / PPG / polybutylène Glycol-8/5/3 Glycérine de NOF vendu sous la dénomination commerciale Wilbride^{®}S753 ou encore le glyceryl-polymethacrylate de Sederma vendu sous la dénomination commerciale Lubragel^{®}MS; la triméthylglycine vendu sous la dénomination commerciale Aminocoat^{®} par la société Ashahi Kasei Chemicals et divers extraits de plantes tels que des extraits de Castanea sativa, des protéines de noisette hydrolysées, les polysaccharides de Tuberosa Polyanthes, l'huile de noyau d'Argania spinosa et les extraits de nacre contenant un conchyoline qui sont vendus notamment par la compagnie Maruzen (Japon) sous le nom commercial Pearl Extract®.

D'autres exemples d'agents hydratants incluent les composés stimulant l'expression de la matriptase MT/SP1, tel qu'un extrait de pulpe de caroube, ainsi que les agents stimulant l'expression de FN3K; les agents augmentant la prolifération ou la différenciation des kératinocytes tels que les extraits de *Thermus thermophilus* ou de fleur de *Camellia Japonica Alba Plena* ou de coques de fèves de *Theobroma cacao,* les extraits hydrosolubles de maïs, les extraits peptidiques de *Voandzeia subterranea* et le niacinamide ; les lipides épidermiques et les agents augmentant la synthèse de lipides épidermiques, soit directement, soit en stimulant certaines β-glucosidases qui modulent la déglycosylation de précurseurs lipidiques comme le glucosylcéramide en céramides, tels que les phospholipides, les céramides, les hydrolysats de protéine de lupin.

Des exemples d'agents anti-pollution incluent l'extrait de graines de Moringa pterygosperma (par exemple le Purisoft^{®} de LSN); l'extrait de beurre de karité (par exemple Detoxyl^{®} de Silab), un mélange d'extrait de lierre, d'acide phytique, d'extrait de graine de tournesol (par exemple l'Osmopur^{®} de Sederma).

Des exemples d'agents kératolytiques incluent les α-hydroxyacides (par exemple les acides glycolique, lactique, citrique, malique, mandélique, ou tartrique) et les β-hydroxyacides (par exemple l'acide salicylique), et leurs esters, tels que les C12-13 alkyl lactates, et les extraits de plantes contenant ces hydroxyacides, tels que des extraits d'Hibiscus sabdriffa.

Des exemples d'agents anti-inflammatoires incluent le bisabolol, l'allantoïne, l'acide tranexamique, l'oxyde de zinc, l'oxyde de soufre et ses dérivés, le sulfate de chondroïtine, l'acide glycyrrhizinique et ses dérivés tels que les glycyrrhizinates.

Des exemples d'astringents incluent les extraits d'hamamélis.

Des exemples d'agents blanchissants incluent l'arbutine et ses dérivés, l'acide férulique (tel que le Cytovector® : eau, glycol, lécithine, acide férulique, hydroxyéthylcellulose, commercialisé par BASF) et ses dérivés, l'acide kojique, le résorcinol, l'acide lipoïque et ses dérivés tel que le monolipoate de resvératrol diacétate tel que décrit dans la demande de brevet WO2006134282, l'acide ellagique, le leucodopachrome et ses dérivés, la vitamine B3, l'acide linoléique et ses dérivés, les céramides et leurs homologues, un peptide tel que décrit dans la demande de brevet WO2009010356, un bioprécurseur tel que décrit dans la demande de brevet WO2006134282 ou un sel de tranexamate tel que le sel de chlorhydrate de tranexamate cétylique, un extrait de réglisse (extrait de Glycyrrhiza glabra), qui est vendu notamment par la société Maruzen sous le nom commercial Licorice extract®, un agent blanchissant ayant également un effet antioxydant, comme les composés de vitamine C, y compris les sels d'ascorbate, les esters ascorbyle d'acides gras ou d'acide sorbique, et d'autres dérivés de l'acide ascorbique, par exemple, les phosphates d'ascorbyle, tels que le magnésium ascorbyl phosphate et le sodium ascorbyl phosphate, ou les esters de saccharide d'acide ascorbique, qui incluent, par exemple, l'ascorbyle-2-glucoside, le L-ascorbate de 2-O-alpha-D-glucopyranosyle, ou le L-ascorbate de 6-O-bêta-D-galactopyranosyle. Un agent actif de ce type est vendu en particulier par la société DKSH sous le nom commercial Ascorbyl glucoside ®.

Des exemples d'agents favorisant la microcirculation incluent un extrait de lupin (tel que l'Eclaline^{®} de Silab), de ruscus, de marron d'inde, de lierre, de ginseng ou de mélilot, la caféine, le nicotinate et ses dérivés, un extrait d'algue de Corallina officinalis tel que celui commercialisé par CODIF ; et leurs mélanges. Ces agents actifs sur la microcirculation cutanée peuvent être utilisés pour éviter le ternissement du teint et/ou améliorer l'homogénéisation et l'éclat du teint.

La composition utilisée selon l'invention peut en outre comprendre en plus du polypeptide selon l'invention, au moins un actif choisi parmi : les agents stimulant l'expression de la tensine 1 tel qu'un extrait d'élémi; les agents stimulant l'expression de la FN3K et/ou de la FN3K RP tel que un extrait de *Butea frondosa ;* les agents stimulant l'expression de CERT ou d'ARNT2; les agents stimulant la production de facteurs de croissance; les agents anti-glycation ou déglycants; les agents augmentant la synthèse de collagène ou prévenant sa dégradation (agents anti-collagénases, notamment inhibiteurs de métalloprotéinases matricielles) en particulier les agents augmentant la synthèse de collagène IV et/ou de hyaluronane et/ou de fibronectine, tel qu'au moins un oligopeptide acylé, notamment celui commercialisé par la société SEDERMA sous la dénomination commerciale Matrixyl^{®} 3000; les agents augmentant la synthèse d'élastine ou prévenant sa dégradation (agents anti-élastases); les agents augmentant la synthèse de glycosaminoglycanes ou de protéoglycanes ou prévenant leur dégradation (agents anti-protéoglycanases) tel que l'actif Epidermosil^{®} (acide hyaluronique associé au méthylsilanetriol) commercialisé par la société Exsymol; les agents stimulant la synthèse d'intégrines par les fibroblastes ; les agents augmentant la prolifération des fibloblastes; les agents facilitant l'absorption percutanée tels que les alcools, les alcools gras et les acides gras et leurs dérivés ester ou éther, les pyrrolidones, les 4-alkyl-oxazolidin-2-ones telle que la 4-decyloxazolidin-2-one ; les terpènes, les huiles essentielles et les α-hydroxyacides; et leurs mélanges, sans que cette liste ne soit limitative.

L'invention sera maintenant illustrée par les exemples non limitatifs suivants.

### EXEMPLES

### Exemple 1 : Clonage de l'ADN complémentaire codant pour LCE6A et synthèse par génie génétique du polypeptide LCE6A

La Demanderesse a cloné l'ADN complémentaire (ADNc) codant pour LCE6A humaine, et a produit sous forme recombinante bactérienne la protéine LCE6A en fusion avec la glutathion S transférase (GST).

### Protocole :

### 1- Clonage de l'ADN complémentaire codant pour LCE6A humaine

La Demanderesse a obtenu des ADNc produits à partir d'une fraction enrichie en kératinocytes granuleux humains, comme décrit dans des travaux précédemment publiés (Toulza et al., Large scale identification of human genes implicated in epidermal barrier function, Genome Biology 2007, 8 :R107). Ces ADNc ont été utilisés pour cloner l'ADNc codant pour LCE6A par amplification en chaîne par polymérase (en anglais PCR pour « polymerase chain reaction ») à l'aide des amorces 5'atgtcacagcagaagcagca3'et 5'gtcgccttcacactctcctc3'. Le produit de PCR, d'une taille de 240 paires de base, a été introduit dans le vecteur pCR®2.1-TOPO® en utilisant le kit de clonage TOPO TA Cloning® (Invitrogen), selon les instructions du fabriquant. Un clone positif a été sélectionné et nommé pCR2.1-TOPO-LCE6A. Le plasmide pCR2.1-TOPO-LCE6A a été digéré par l'enzyme de restriction EcoRI, et le fragment correspondant à l'ADNc LCE6A de 258 paires de bases a été purifié et sous-cloné dans le vecteur d'expression procaryote pGEX6P1 (GE Healthcare) digéré par EcoRI. Le criblage orienté des clones obtenus a permis de sélectionner un clone nommé pGEX6P1-LCE6A.

### 2- Synthèse par génie génétique du polypeptide LCE6A

Des bactéries E. coli BL21-CodonPlus®-DE3-RIL (Stratagene) ont été transformées par le plasmide pGEX6P1-LCE6A et la production de la protéine LCE6A recombinante a été réalisée comme suit : les bactéries transformées ont été cultivées une nuit sous agitation à 250 rotations par minute(rpm) à 37°C dans 10 ml de milieu LB-ampicilline-chloramphénicol (10 g/l NaCl, 10 g/l tryptone, 8 g/l extrait de levure, 100 mg/l ampicilline, 50 mg/ml chloramphénicol, pH7). Cette culture est utilisée le lendemain pour ensemencer 500 ml de milieu LB-ampicilline-chloramphénicol et poursuivre la culture pendant environ 2h, jusqu'à obtenir une densité optique à 600 nm de la culture comprise entre 0,6 et 0,8. La production de la protéine recombinante LCE6A en fusion avec la glutathion-S-transférase (GST) à son extrémité N-terminale (dénommée ci-après GST-LCE6A) est alors induite par prolongation de la culture pendant 4h en présence de 0,1 mM isopropyl thio-β-D-galactoside (IPTG). La culture bactérienne est ensuite placée sur glace pendant 10 minutes, puis centrifugée 10 minutes à 6000 rpm à +4°C. Après élimination du surnageant, le culot est stocké au minimum 12h à -20°C.

Le lysat bactérien est obtenu comme suit : le culot bactérien est suspendu dans 50 ml de tampon de solubilisation (20 mM Tris, 150 mM NaCl, 1 mM EDTA, additionné de 100 µl de cocktail d'inhibiteurs de protéases bactériennes (P8465, Sigma)), puis centrifugé 10 minutes à 13000 rpm +4°C. Le surnageant est éliminé, et le culot est suspendu dans 50 ml de tampon de solubilisation. On ajoute 5 ml de lysosyme à 100 mg/ml et on incube 15 minutes sur glace. On ajoute ensuite 500 µl de dithiothréitol 1 M et 7,75 ml de sarkosyl 10%, on mélange par inversion et la suspension est soniquée (ultrasonic cell disruptor XL2000, Misonix) 3 fois 20 secondes avec retour sur glace 5 minutes entre chaque sonication. Le lysat est ensuite centrifugé 30 minutes à 10 000 rpm +4°C. Le surnageant est récupéré et additionné de 20 ml de Triton-X-100 à 10% et de 16,75 ml de tampon de solubilisation. La lyse est achevée par une incubation de cette solution pendant 30 minutes sous agitation douce à température ambiante, puis le lysat est filtré (pores de 0,45 µm de diamètre).

GST-LCE6A est purifiée à partir de ce lysat bactérien par affinité sur colonne « glutathione-sépharose 4 fast flow » (2,5 ml de matrice) (GE-Healthcare Amersham) selon les instructions du fabriquant. Le filtrat est déposé sur la colonne et la fraction non retenue est éliminée. La colonne est rincée par 40 ml de tampon « phosphate buffered saline » (PBS) (1,47 mM KH₂PO₄, 4,3 mM Na₂HPO₄, 137 mM NaCl, 2,7 mM KCl, 0,9 mM CaCl₂, pH 7,4) et l'élution est réalisée en déposant 8 ml de glutathion à 10 mM en tampon Tris 50 mM pH8, puis 4 ml de PBS. Douze fractions d'élution de 1 ml sont recueillies en sortie de colonne. Un aliquot de 12,5 µl de chacune de ces fractions est séparé par électrophorèse en condition dénaturante SDS-PAGE 15% (« SDS-polyacrylamide gel electrophoresis ») et transféré sur membrane de nitrocellulose par électrotransfert. Cette membrane est ensuite utilisée pour tester par la méthode dite de « Western Blot » (immunotransfert) la présence de la protéine recombinante dans les fractions d'élution. L'anticorps primaire est un anticorps monoclonal reconnaissant la GST (mouse mAb 26H1, Cell Signaling Technology) utilisé au 1/10000^{e}, l'anticorps secondaire couplé à la peroxydase (Horseradish peroxydase conjugated-goat anti-mouse IgG(H+L), Zymed) est utilisé au 1/10000^{e} et la révélation est réalisée avec le réactif ECL (Amersham Pharmacia Biotech). Les fractions d'élution contenant la protéine recombinante bactérienne sont regroupées, dialysées contre 1000 volume de PBS à l'aide de boudins de dialyse (MWCO 3500) une nuit à +4°C sous agitation. Le dialysat est ensuite dosé par la méthode Bradford à l'aide du kit « BioRad protein assay » (BioRad).

Le rendement est en moyenne de 3 mg de protéine recombinante pour 500 ml de culture bactérienne.

### Exemple 2 : Etude de l'expression et de la localisation de la protéine LCE6A dans l'épiderme humain normal

### Protocole :

### 1. Production et purification d'antisérum de lapin reconnaissant spécifiquement la protéine LCE6A :

Après analyse de la séquence primaire de la protéine LCE6A, la Demanderesse a choisi le polypeptide de 15 acides aminés CHSSSQRPEVQKPRR, correspondant aux résidus 42 à 56 de LCE6A, non homologue aux autres protéines de la famille LCE, désigné ci-après SEQ ID N°6. Ce polypeptide a été produit et utilisé pour immuniser deux lapins (4 injections successives à la semaine 1, 4, 7 et 9). L'antisérum est prélevé la 11^{ème} semaine. Ces étapes ont été réalisées par la société Génosphère Biotechnologies. Le sérum anti-peptide a ensuite été purifié par affinité par la Demanderesse. Pour cela, le polypeptide correspondant à la séquence d'acides aminés SEQ ID N°6 a été immobilisé sur billes d'agarose à l'aide du kit Sulfolink® (Pierce). Les billes sont incubées avec 10 ml d'antisérum dilué au ½ dans du tampon de charge « Gentle Ag/Ab binding buffer » (Pierce) pendant 1h à température ambiante sous agitation douce. Les billes sont sédimentées sur colonne et le liquide non retenu est éliminé. La colonne est rincée par 25 ml de tampon de charge additionné de 0,5 M NaCl, puis 5 ml de tampon de charge. Le sérum anti-peptide LCE6A est élué par dépôt sur la colonne de 9 ml de tampon d'élution « Gentle Ag/Ab elution buffer » (Pierce), et la réactivité de chacune des 9 fractions d'élution est testée par « Western Blot » sur la protéine recombinante GST-LCE6A. L'incubation est réalisée avec un aliquot de chacune des fractions d'élution dilué au 1/50^{e}, puis avec un anticorps secondaire anti-immunoglobuline de lapin couplé à la péroxidase dilué au 1/10000^{e} (Zymed). La révélation est réalisée avec le kit ECL (Amersham Pharmacia Biotech). Les fractions présentant la plus forte réactivité contre la protéine recombinante sont regroupées et dialysées contre 1000 volumes de tampon TBS (0,05 M Tris, 0,15 M NaCl, pH 7,6) à l'aide de boudins de dialyse (MWCO 3500) une nuit à +4°C sous agitation. Le dialysat est concentré environ 20 fois par ultrafiltration (Vivaspin 15R, 30000 MWCO, Vivascience). Le dialysat concentré, correspondant à l'antisérum purifié, est titré sur la protéine recombinante par « Western Blot ». Il reconnaît 100 ng de GST-LCE6A jusqu'à une dilution de 1/2000^{e}.

### 2. Détection de la protéine LCE6A par immunohistologie :

L'expression et la localisation de la protéine LCE6A dans l'épiderme humain normal a été analysée par immunohistochimie et immunofluorescence à l'aide du sérum anti-peptide LCE6A. Des échantillons de peau abdominale humaine normale ont été fixés dans du formol pendant 24h et inclus en paraffine. L'immunodétection est réalisée sur les coupes après démasquage de l'antigène par incubation pendant 40 minutes dans 50 mM glycine-HCl pH 3,5 à 95°C.

Dans les expériences d'immunohistochimie, l'immunodétection est réalisée avec le kit « Impress rabbit » (Vector Laboratories) selon les instructions du fabriquant, en utilisant le sérum anti-peptide LCE6A dilué au 1/250^{e}.

Dans les expériences d'immunofluorescence, un double marquage a été réalisé à l'aide du sérum anti-peptide LCE6A dilué au 1/100^{e} et d'un anticorps monoclonal de souris dirigé contre la (pro)filaggrine (AHF3, Simon et al., J Invest Dermatol 1995, 105 :432) dilué au 1/1000^{e}. Les anticorps secondaires sont des immunoglobulines anti-immunoglobuline de souris couplées à l'AlexaFluor488 et des immunoglobulines anti-immunoglobuline de lapin couplées à l'AlexaFluor555 (Invitrogen), utilisés au 1/1000e.

### Résultats :

Le marquage obtenu par immunohistochimie ou immunofluorescence avec le sérum anti-peptide LCE6A apparaît tardivement au cours de la différenciation épidermique, à la transition couche granuleuse / couche cornée et dans la partie basse de la couche cornée. La comparaison des marquages obtenus avec le sérum anti-peptide LCE6A et l'anticorps monoclonal anti-(pro)filaggrine en immunofluorescence a permis de mettre en évidence que les deux protéines, LCE6A et filaggrine, sont exprimées au niveau de la couche granuleuse et du bas de la couche cornée de l'épiderme humain.

### Exemple 3 : Etude de l'expression et de la localisation de la protéine LCE6A dans l'épiderme humain psoriasique

### Protocole :

### 1. Antisérum de lapin reconnaissant spécifiquement la protéine LCE6A :

L'antisérum de lapin reconnaissant spécifiquement la protéine LCE6A humaine utilisé est le même que celui dont la production et la purification ont été décrites dans l'exemple 2.

### 2. Détection de la protéine LCE6A par immunohistologie :

L'expression et la localisation de la protéine LCE6A dans l'épiderme humain normal a été analysée par immunofluorescence indirecte à l'aide du sérum anti-peptide LCE6A. Les échantillons proviennent de peau abdominale humaine normale ou de lésions psoriasiques prélevées sur 15 individus atteints de psoriasis. Ces échantillons de peau ont été cryofixés et conservés à - 80°C. Des cryocoupes ont été réalisées, séchées à l'air libre environ 2 heures, fixées à l'acétone 10 minutes, puis conservées à -80°C. L'immunodétection est réalisée par immunofluorescence sur les coupes après démasquage de l'antigène par incubation pendant 20 minutes dans la solution « target retrieval solution pH9 » (Dako) à 95°C. Le sérum anti-peptide LCE6A est utilisé à une dilution de 1/250^{e}. L'anticorps secondaire est une immunoglobuline anti-immunoglobuline de lapin couplée à l'AlexaFluor555 (Invitrogen), utilisée au 1/1000^{e}.

### Résultats :

L'observation des coupes de peaux lésionnelles provenant des 15 individus atteints de psoriasis montre une baisse importante de l'expression de LCE6A dans la couche granuleuse et le bas de la couche cornée de l'épiderme, en comparaison à la peau normale. Il semble donc que cette pathologie, qui présente une profonde perturbation de la prolifération et de la différenciation kératinocytaires, ainsi qu'un trouble important de la fonction barrière de l'épiderme, présente une quantité de protéine LCE6A, exprimée par le kératinocyte granuleux puis incorporée à l'enveloppe cornée, considérablement réduite.

### Exemple 4 : Caractérisation biochimique et fonctionnelle de LCE6A - Test de liaison aux transglutaminases

La Demanderesse a réalisé des tests de liaison aux transglutaminases in vitro à l'aide de LCE6A recombinante possédant une étiquette Histidine C-terminale (LCE-His).

### 1. Clonage de la protéine LCE6A-His et purification :

### Protocole :

Pour cloner l'ADNc codant pour LCE6A-His, l'ADNc LCE6A a été amplifié par PCR à partir du vecteur pGEX6P1 - LCE6A à l'aide des amorces 5'catatgtcacagcagaagcagcaa3' et 5'ctcgaggtcgccttcacactc3'. Le produit de PCR, d'une taille de 248 paires de base, a été introduit dans le vecteur pCR®2.1-TOPO® en utilisant le kit de clonage TOPO TA Cloning® (Invitrogen), selon les instructions du fabriquant. Un clone positif a été sélectionné et nommé pCR2.1-TOPO-LCE6A-NdeI-XhoI. Ce plasmide a été digéré par les enzymes de restriction NdeI et XhoI, et le fragment correspondant à l'ADNc LCE6A de 248 paires de bases a été purifié et sous-cloné dans le vecteur d'expression procaryote pET41b (Novagen) digéré par NdeI et XhoI. Le criblage des clones obtenus a permis de sélectionner un clone nommé pET41b-LCE6A-His.

Des bactéries E. coli BL21-CodonPlus®-DE3-RIL (Stratagene) ont été transformées par le plasmide pET41b-LCE6A-His et la production de la protéine recombinante a été réalisée comme suit : les bactéries transformées ont été cultivées une nuit sous agitation à 250 rpm à 37°C dans 10 ml de milieu LB-kanamycine-chloramphénicol (10 g/l NaCl, 10 g/l tryptone, 8 g/l extrait de levure, 50 mg/l kanamycine, 50 mg/l chloramphénicol, pH7). Cette culture est utilisée le lendemain pour ensemencer 500 ml de milieu LB-kanamycine-chloramphénicol et poursuivre la culture pendant environ 2h, jusqu'à obtenir une densité optique à 600 nm de la culture comprise entre 0,6 et 0,8. La production de la protéine recombinante LCE6A-His est alors induite par prolongation de la culture pendant 4h en présence de 0,1 mM isopropyl thio-β-D-galactoside (IPTG). La culture bactérienne est ensuite placée sur glace pendant 10 minutes, puis centrifugée 10 minutes à 6000 rpm à +4°C. Après élimination du surnageant, le culot est stocké au minimum 12h à -20°C.

Le lysat bactérien est obtenu comme suit : le culot bactérien est suspendu dans 100 ml de tampon de lyse (50 mM NaH₂PO4, 300 mM NaCl, 10 mM imidazole, pH8, 0,1 mg/ml lyzozyme, additionné de 100 µl de cocktail d'inhibiteurs de protéases bactériennes (P8465, Sigma)) et incubé 1h30 sous agitation douce à +4°C. Le lysat est soniqué (ultrasonic cell disruptor XL2000, Misonix) 6 fois 5 secondes avec retour sur glace 5 minutes entre chaque sonication, puis centrifugé 30 minutes à 14000 rpm +4°C. Le surnageant est récupéré et filtré (pores de 0,45 µm de diamètre) avant purification par affinité sur colonne de nickel (1 ml de matrice) « His-Trap High Performance » (Amersham Biosciences). La colonne est équilibrée par 10 volumes de tampon de lyse, puis le lysat est chargé. Le liquide non retenu est éliminé et la colonne est rincée par 15 volumes de tampon de lavage (50 mM NaH₂PO4, 300 mM NaCl, 20 mM imidazole, pH8). L'élution est réalisée avec 10 volumes de tampon d'élution (50 mM NaH₂PO4, 300 mM NaCl, 0,5 M imidazole, pH8). La présence de la protéine recombinante dans les fractions d'élution est détectée par « Western Blot ». L'anticorps primaire est un anticorps monoclonal anti-tétra-His (mouse monoclonal IgG1 « Tetra-His antibody », Qiagen) utilisé au 1/2000^{e}, l'anticorps secondaire couplé à la peroxydase (Horseradish peroxydase conjugated-goat anti-mouse IgG(H+L), Zymed) est utilisé au 1/10000^{e} et la révélation est réalisée avec le réactif ECL (Amersham Pharmacia Biotech). Les fractions d'élution contenant la protéine recombinante bactérienne sont regroupées et dialysées contre 1000 volumes de PBS à l'aide de boudins de dialyse (MWCO 3500) une nuit à +4°C sous agitation. Le dialysat est ensuite dosé par la méthode Bradford à l'aide du kit « BioRad protein assay » (BioRad). Le rendement est en moyenne de 3 mg de protéine recombinante pour 500 ml de culture bactérienne.

### 2. Test in vitro de liaison à la transglutaminase 2 (TGM2):

### Protocole :

Un test de liaison de LCE6A par la transglutaminase 2 (transglutaminase purifiée de foie de cobaye, Sigma T5398) a été réalisé in vitro.

Dans une première expérience, la Demanderesse a testé la capacité de la TGM2 à former des pontages entre la LCE-His et une molécule porteuse d'une liaison amine, la monodansyl-cadavérine (MDC). Pour cela, la TGM2 (10 ng/µL) a été incubée en présence de 0,35 µg de LCE6A-His en solution dans un tampon 50 mM Tris, 100 mM DTT, pH 7,4, additionné de 10 mM CaCl₂ et 500 µM MDC (Fluka). Après incubation 2h ou 18h à 37°C, la réaction est arrêtée par ajout d'EDTA à 25 mM final. Le contrôle négatif correspond à la même expérience réalisée en l'absence de CaCl₂ et en présence de 100 mM d'ETDA après incubation de 18h. Le produit de la réaction est séparé par SDS-PAGE 15%, puis visualisé par illumination aux UV.

Dans une seconde expérience, la Demanderesse a également testé la capacité de la TGM2 à former des pontages de la LCE6A avec elle-même. Pour cela, la TGM2 (10 ng/µL) a été incubée en présence de 0,35 µg de LCE6A-His dans un tampon 50 mM Tris, 100 mM DTT, pH 7,4, additionné de 10 mM CaCl₂. Après incubation 2h ou 18h à 37°C, la réaction est arrêtée par ajout d'EDTA à 25 mM final. Le contrôle négatif correspond à la même expérience réalisée en l'absence de CaCl₂ et en présence de 100 mM d'ETDA après incubation de 18h. Le produit de la réaction est séparé par SDS-PAGE 15% et transféré sur membrane de nitrocellulose. Les liens intermoléculaires sont visualisés par « Western Blot » comme décrit précédemment avec un anticorps anti-His (mouse monoclonal IgG1 « Tetra-His antibody », Qiagen).

### Résultats :

Dans la première expérience, il a été détecté dès 2h d'incubation plusieurs bandes fluorescentes migrant à la hauteur de la MDC libre (front de migration), ou de LCE6A-His (environ 17 kDa). D'autres bandes de plus haut poids moléculaire, de faible intensité, migrent à la taille de dimères ou de multimères de LCE6A-His. Aucune bande fluorescente, hormis celle correspondant à la MDC libre, n'est détectée lorsque la même expérience est réalisée en présence de 100 mM EDTA et en absence de calcium.

Dans la deuxième expérience, le résultat du « Western Blot » montre une bande d'environ 17 kDa correspondant à LCE6A-His monomérique, et plusieurs bandes de plus haut poids moléculaire migrant à une taille équivalente à des dimères ou des multimères de LCE6A-His. Seule la bande correspondant à LCE6A-His monomérique est détectée lorsque l'expérience est réalisée dans les mêmes conditions mais en absence de calcium et en présence de 100 mM d'EDTA.

Par conséquent, ces expériences permettent de confirmer que la protéine LCE6A est bien un substrat de la transglutaminase 2. LCE6A se comporte à la fois comme donneur et un accepteur de résidus d'acides aminés nécessaires à la liaison ε-(γ-glutamnyl)lysyl avec la transglutaminase.

### 3. Test in vitro de liaison à la transglutaminase 3 (TGM3) :

Un test de liaison de LCE6A par la transglutaminase 3 (tranglutaminase 3 humaine recombinante, R&D Systems) a été réalisé in vitro. Ces tests sont réalisés selon le même protocole expérimental que celui décrit dans l'exemple 4 paragraphe 2 avec la transglutaminase 2.

Dans une première expérience, la Demanderesse a testé la capacité de la TGM3 à former des pontages entre la LCE-His et la monodansyl-cadavérine (MDC). Pour cela, la TGM3 (10 ng/µL) a été incubée en présence de 0,35 µg de LCE6A-His en solution dans un tampon 50 mM Tris, 100 mM DTT, pH 7,4, additionné de 10 mM CaCl₂ et 500 µM MDC (Fluka). Après incubation 2h ou 18h à 37°C, la réaction est arrêtée par ajout d'EDTA à 25 mM final. Le contrôle négatif correspond à la même expérience réalisée en l'absence de CaCl₂ et en présence de 100 mM d'ETDA après incubation de 18h. Le produit de la réaction est séparé par SDS-PAGE 15%, puis visualisé par illumination aux UV.

Dans une seconde expérience, la Demanderesse a testé la capacité de la TGM3 à former des pontages de la LCE6A avec elle-même. Pour cela, la TGM3 (10 ng/µL) a été incubée en présence de 0,35 µg de LCE6A-His dans un tampon 50 mM Tris, 100 mM DTT, pH 7,4, additionné de 10 mM CaCl₂. Après incubation 2h ou 18h à 37°C, la réaction est arrêtée par ajout d'EDTA à 25 mM final. Le contrôle négatif correspond à la même expérience réalisée en l'absence de CaCl₂ et en présence de 100 mM d'ETDA après incubation de 18h. Le produit de la réaction est séparé par SDS-PAGE 15% et transféré sur membrane de nitrocellulose. Les liens intermoléculaires sont visualisés par Western Blot comme décrit précédemment avec un anticorps anti-His (mouse monoclonal IgG1 « Tetra-His antibody », Qiagen).

### Résultats :

Dans la première expérience, il a été détecté dès 2h d'incubation plusieurs bandes fluorescentes migrant à la hauteur de la MDC libre (front de migration), ou de LCE6A-His (environ 17 kDa). D'autres bandes de plus haut poids moléculaire, de faible intensité, migrent à la taille de dimères ou de multimères de LCE6A-His. Aucune bande fluorescente, hormis celle correspondant à la MDC libre, n'est détectée lorsque la même expérience est réalisée en présence de 100 mM EDTA et en absence de calcium.

Dans la deuxième expérience, le résultat du Western Blot montre une bande d'environ 17 kDa correspondant à LCE6A-His monomérique, et plusieurs bandes de plus haut poids moléculaire migrant à une taille équivalente à des dimères ou des multimères de LCE6A-His. Seule la bande correspondant à LCE6A-His monomérique est détectée lorsque l'expérience est réalisée dans les mêmes conditions mais en absence de calcium et en présence de 100 mM d'EDTA.

Par conséquent, ces expériences permettent de confirmer que la protéine LCE6A est bien un substrat de la transglutaminase 3. LCE6A se comporte à la fois comme donneur et un accepteur de résidus d'acides aminés nécessaires à la liaison ε-(γ-glutamnyl)lysyl avec la transglutaminase. Les transglutaminases 1, 3 et 5, qui catalysent la même réaction que la TGM2, sont impliquées in vivo dans la formation de la couche cornée et la formation des liens isopeptidiques nécessaires à la résistance et l'insolubilité de l'enveloppe cornée. LCE6A est donc, in vitro, le substrat d'au moins une des transglutaminases impliquées, in vivo, dans la formation de l'enveloppe cornée.

### Exemple 5 : Caractérisation de la séquence peptidique de LCE6A impliquée dans les liaisons à la transglutaminase

### Protocole :

Pour déterminer quelle région de la protéine LCE6A est nécessaire à la liaison par les transglutaminases, la Demanderesse a fait synthétiser par la société MilleGen différents polypeptides, biotinylés à leur extrémité N-terminale, d'une longueur de 9 à 13 acides aminés, correspondant à différentes régions de la séquence d'acides aminés de la LCE6A.

Ces polypeptides ont été utilisés dans des tests de liaison in situ. Des cryocoupes de peau abdominale humaine, saturées par incubation en présence d'albumine bovine à 1% en PBS pendant 30 minutes à température ambiante, ont été mises en présence de 100 mM Tris (pH 7,4) contenant 100 µM de polypeptide biotinylé et 5 mM de CaCL₂. Après une incubation de 2h à température ambiante, la réaction est arrêtée par ajout d'EDTA à 25 mM pendant 5 minutes. Les coupes sont ensuite incubées dans du PBS contenant 1% de SDS pour détruire les liens non covalents.

La présence de polypeptides biotinylés liés aux enveloppes cornées par les transglutaminases présentes et actives dans le tissus est détectée par incubation des coupes avec de la streptavidine-AlexaFluor555 à 5 µg/ml (Invitrogen) et visualisation au microscope confocal.

Un contrôle positif est réalisé dans une expérience similaire mais en incubant les coupes avec 100 µM de cadavérine couplée à l'AlexaFluor555 (Invitrogen) à la place des polypeptides. Pour les contrôles négatifs, les cryocoupes sont incubées avec chaque polypeptide biotinylé ou avec la cadavérine couplée à l'AlexaFluor555 mais l'incubation se fait en présence de 100 mM d'EDTA et en absence de calcium.

### Résultats :

Le contrôle positif montre un marquage du contour des kératinocytes granuleux, correspondant à la région de la coupe où se trouvent des transglutaminases actives. Les polypeptides correspondants à la séquence d'acides aminés SEQ ID N°3, 4 et 5 montrent un marquage similaire à celui obtenu après incubation avec la cadavérine-AlexaFluor555, alors qu'aucun marquage n'est obtenu lorsque ces mêmes polypeptides ou la cadavérine-AlexaFluor555 sont incubés en présence d'EDTA et en absence de calcium. L'incubation des coupes en présence de calcium avec le polypeptide RPAPPPISGGGYRAR, dont la séquence ne correspond à aucun fragment de LCE6A, ne donne aucun marquage péricellulaire des kératinocytes de la couche granuleuse. Enfin, le polypeptide MSQAKQQSW (SEQ ID N°7) correspondant à la SEQ ID N°3 mutée, et le polypeptide EVAKPRRARQALR (SEQ ID N°8) correspondant à la SEQ ID N°5 mutée, ne donne aucun marquage péricellulaire des kératinocytes de la couche granuleuse.

Par conséquent, les séquences d'acides aminés SEQ ID N°3, 4 et 5 renferment les résidus nécessaires aux transglutaminases présentes dans la couche granuleuse de l'épiderme humain pour établir des liens covalents avec l'enveloppe cornée. La perte de capacité à lier les enveloppes cornées des polypeptides correspondant aux SEQ ID N°1 et N°3 mutées suggère que l'enchaînement d'un résidu glutamine (Q) et d'un résidu Lysine (K) dans la séquence de la protéine LCE6A est nécessaire pour permettre cette liaison.

### Exemple 6 : Composition cosmétique (sérum H/E)

La composition suivante peut être préparée de façon classique pour l'homme du métier. Les quantités indiquées ci-dessous sont exprimées en pourcentages pondéraux. Les ingrédients en majuscules sont identifiés conformément à la dénomination INCI.

| **Nom INCI** | **% (poids/poids)** |
|---|---|
| Eau | QSP 100,00 |
| Agent chélatant | 0,05 |
| Ajusteur de pH | 0,05 |
| Conservateur | 0,05 |
| Glycol | 3,25 |
| AMMONIUM ACRYLOYLDIMETHYLTAURATE/VP COPOLYMER | 1,20 |
| ACRYLATES/C10-30 ALKYL ACRYLATE CROSSPOLYMER | 0,20 |
| GLYCERINE | 3,00 |
| GLYCERYLPOLYMETHACRYLATE | 4.18 |
| SODIUM ACETYLATED HYALURONATE | 0, 05 |
| Huile | 10, 00 |
| ALCOOL | 8,00 |
| PARFUMS | 0,30 |
| Polypeptide SEQ ID N°4 obtenu selon l'exemple 4 | 0,05 |

Cette composition peut être appliquée quotidiennement, matin et/ou soir, sur des peaux particulièrement déshydratées et/ou exposées aux agressions de l'environnement, pour améliorer leur confort et uniformiser le teint.

## Revendications

1. Utilisation cosmétique d'une quantité efficace d'un polypeptide isolé, naturel ou synthétique, appartenant à la famille des protéines tardives de l'enveloppe cornée (LCE), ledit polypeptide étant choisi parmi :
- les fragments de la séquence SEQ ID N°2 de longueur d'au moins 5 acides aminés consécutifs, lesdits fragments présentant au moins un domaine glutamine (Gln) - lysine (Lys) servant de substrat aux transglutaminases ; et
- les analogues desdits fragments de la séquence SEQ ID N°2 présentant au moins 80% d'homologie avec un desdits fragments de la séquence SEQ ID N°2, lesdits analogues présentant au moins un domaine glutamine (Gln) - lysine (Lys) servant de substrat aux transglutaminases,
pour prévenir et/ou traiter les signes de la sècheresse cutanée.

2. Utilisation cosmétique d'une quantité efficace d'un polypeptide isolé, naturel ou synthétique, appartenant à la famille des protéines tardives de l'enveloppe cornée (LCE), ledit polypeptide étant choisi parmi :
- les fragments de la séquence SEQ ID N°2 de longueur d'au moins 5 acides aminés consécutifs, lesdits fragments présentant au moins un domaine glutamine (Gln) - lysine (Lys) de la séquence SEQ ID N°2, lesdits fragments présentant au moins un domaine glutamine (Gln) - lysine (Lys) servant de substrat aux transglutaminases ; et
- les analogues desdits fragments de la séquence SEQ ID N°2 présentant au moins 80% d'homologie avec un desdits fragments de la séquence SEQ ID N°2, lesdits analogues présentant au moins un domaine glutamine (Gln) - lysine (Lys) servant de substrat aux transglutaminases,
pour prévenir et/ou traiter les signes du vieillissement cutané.

3. Polypeptide isolé, naturel ou synthétique, appartenant à la famille des protéines tardives de l'enveloppe cornée (LCE), ledit polypeptide étant choisi parmi :
- les fragments de la séquence SEQ ID N°2 de longueur d'au moins 5 acides aminés consécutifs, lesdits fragments présentant au moins un domaine glutamine (Gln) - lysine (Lys) de la séquence SEQ ID N°2, lesdits fragments présentant au moins un domaine glutamine (Gln) - lysine (Lys) servant de substrat aux transglutaminases ; et
- les analogues desdits fragments de la séquence SEQ ID N°2 présentant au moins 80% d'homologie avec un desdits fragments de la séquence SEQ ID N°2, lesdits analogues présentant au moins un domaine glutamine (Gln) - lysine (Lys) servant de substrat aux transglutaminases,
pour son utilisation pour prévenir et/ou traiter les troubles trophiques cutanés ou ceux consécutifs à des troubles de la cicatrisation.

4. Polypeptide isolé, naturel ou synthétique, appartenant à la famille des protéines tardives de l'enveloppe cornée (LCE), ledit polypeptide étant choisi parmi :
- les fragments de la séquence SEQ ID N°2 de longueur d'au moins 5 acides aminés consécutifs, lesdits fragments présentant au moins un domaine glutamine (Gln) - lysine (Lys) de la séquence SEQ ID N°2, lesdits fragments présentant au moins un domaine glutamine (Gln) - lysine (Lys) servant de substrat aux transglutaminases ; et
- les analogues desdits fragments de la séquence SEQ ID N°2 présentant au moins 80% d'homologie avec un desdits fragments de la séquence SEQ ID N°2, lesdits analogues présentant au moins un domaine glutamine (Gln) - lysine (Lys) servant de substrat aux transglutaminases,
pour son utilisation pour prévenir et/ou traiter l'amincissement de l'épiderme et en particulier de la couche cornée et/ou à traiter une fragilité excessive du revêtement cutané et/ou à induire l'épaississement de la couche cornée.

5. Polypeptide isolé, naturel ou synthétique, appartenant à la famille des protéines tardives de l'enveloppe cornée (LCE), ledit polypeptide étant choisi parmi :
- les fragments de la séquence SEQ ID N°2 de longueur d'au moins 5 acides aminés consécutifs, lesdits fragments présentant au moins un domaine glutamine (Gln) - lysine (Lys) de la séquence SEQ ID N°2, lesdits fragments présentant au moins un domaine glutamine (Gln) - lysine (Lys) servant de substrat aux transglutaminases ; et
- les analogues desdits fragments de la séquence SEQ ID N°2 présentant au moins 80% d'homologie avec un desdits fragments de la séquence SEQ ID N°2, lesdits analogues présentant au moins un domaine glutamine (Gln) - lysine (Lys) servant de substrat aux transglutaminases,
pour son utilisation pour traiter l'hyperkératose, la xérose, les ichtyoses, le psoriasis, les lésions tumorales hyperkératosiques bénignes ou malignes ou les kératoses réactionnelles.

6. Utilisation selon la revendication 1 ou 2, ou polypeptide selon l'une des revendications 3 à 5, **caractérisé en ce que** le polypeptide est un fragment de longueur d'au moins 7, de préférence au moins 9, et plus particulièrement au moins 15 acides aminés consécutifs.

7. Utilisation selon la revendication 1 ou 2, ou polypeptide selon l'une des revendications 3 à 5, **caractérisé en ce que** le polypeptide a une séquence choisie parmi SEQ ID N°3, SEQ ID N°4, SEQ ID N°5, et leurs mélanges.

## Patentansprüche

1. Kosmetische Anwendung einer wirksamen Menge eines natürlichen oder synthetischen isolierten Polypeptids, welches zur Familie der späten Proteine der Cornea-Hülle (LCE) gehört, wobei das Polypeptid gewählt ist aus:
- den Fragmenten der Sequenz SEQ ID NO 2 mit einer Länge von mindestens 5 aufeinanderfolgenden Aminosäuren, wobei die Fragmente wenigstens eine Glutamin-Domäne (Gln) aufweisen - wobei Lysin (Lys) als Substrat für die Transglutaminasen dient; und
- den Analogen der Fragmente der Sequenz SEQ ID NO 2, die wenigstens eine 80-prozentige Homologie mit einem der Fragmente der SEQ ID NO 2 aufweisen, wobei die Analoge wenigstens eine Glutamin-Domäne (Gln) aufweisen - wobei Lysin (Lys) als Substrat für die Transglutaminasen dient,
zur Prävention und/oder Behandlung von Zeichen der Hauttrockenheit.

2. Kosmetische Anwendung einer wirksamen Menge eines natürlichen oder synthetischen isolierten Polypeptids, welches zur Familie der späten Proteine der Cornea-Hülle (LCE) gehört, wobei das Polypeptid gewählt ist aus:
- den Fragmenten der Sequenz SEQ ID NO 2 mit einer Länge von mindestens 5 aufeinanderfolgenden Aminosäuren, wobei die Fragmente wenigstens eine Glutamin-Domäne (Gln) aufweisen - Lysin (Lys) der Sequenz SEQ ID NO 2, wobei die Fragmente wenigstens eine Glutamin-Domäne (Gln) aufweisen - wobei Lysin (Lys) als Substrat für die Transglutaminasen dient; und
- den Analogen der Fragmente der Sequenz SEQ ID NO 2, die wenigstens eine 80-prozentige Homologie mit einem der Fragmente der SEQ ID NO 2 aufweisen, wobei die Analoge wenigstens eine Glutamin-Domäne (Gln) aufweisen - wobei Lysin (Lys) als Substrat für die Transglutaminasen dient,
zur Prävention und/oder Behandlung von Zeichen der Hautalterung.

3. Natürliches oder synthetisches isoliertes Polypeptid, welches zur Familie der späten Proteine der Cornea-Hülle (LCE) gehört, wobei das Polypeptid gewählt ist aus:
- den Fragmenten der Sequenz SEQ ID NO 2 mit einer Länge von mindestens 5 aufeinanderfolgenden Aminosäuren, wobei die Fragmente wenigstens eine Glutamin-Domäne (Gln) aufweisen - Lysin (Lys) der Sequenz SEQ ID NO 2, wobei die Fragmente wenigstens eine Glutamin-Domäne (Gln) aufweisen - wobei Lysin (Lys) als Substrat für die Transglutaminasen dient; und
- den Analogen der Fragmente der Sequenz SEQ ID NO 2, die wenigstens eine 80-prozentige Homologie mit einem der Fragmente der SEQ ID NO 2 aufweisen, wobei die Analoge wenigstens eine Glutamin-Domäne (Gln) aufweisen - wobei Lysin (Lys) als Substrat für die Transglutaminasen dient,
zur Anwendung in der Prävention und/oder Behandlung von trophischen Hautstörungen oder Folgen von Wundheilungsstörungen.

4. Natürliches oder synthetisches isoliertes Polypeptid, welches zur Familie der späten Proteine der Cornea-Hülle (LCE) gehört, wobei das Polypeptid gewählt ist aus:
- den Fragmenten der Sequenz SEQ ID NO 2 mit einer Länge von mindestens 5 aufeinanderfolgenden Aminosäuren, wobei die Fragmente wenigstens eine Glutamin-Domäne (Gln) aufweisen - Lysin (Lys) der Sequenz SEQ ID NO 2, wobei die Fragmente wenigstens eine Glutamin-Domäne (Gln) aufweisen - wobei Lysin (Lys) als Substrat für die Transglutaminasen dient; und
- den Analogen der Fragmente der Sequenz SEQ ID NO 2, die wenigstens eine 80-prozentige Homologie mit einem der Fragmente der SEQ ID NO 2 aufweisen, wobei die Analoge wenigstens eine Glutamin-Domäne (Gln) aufweisen - wobei Lysin (Lys) als Substrat für die Transglutaminasen dient,
zur Anwendung in der Prävention und/oder Behandlung einer Verdünnung des Epiderms und insbesondere der Hornhautschicht und/oder Behandlung einer übermäßigen Schwächung der Hautdecke und/oder zur Anregung einer Verdickung der Hornhautschicht.

5. Natürliches oder synthetisches isoliertes Polypeptid, welches zur Familie der späten Proteine der Cornea-Hülle (LCE) gehört, wobei das Polypeptid gewählt ist aus:
- den Fragmenten der Sequenz SEQ ID NO 2 mit einer Länge von mindestens 5 aufeinanderfolgenden Aminosäuren, wobei die Fragmente wenigstens eine Glutamin-Domäne (Gln) aufweisen - Lysin (Lys) der Sequenz SEQ ID NO 2, wobei die Fragmente wenigstens eine Glutamin-Domäne (Gln) aufweisen - wobei Lysin (Lys) als Substrat für die Transglutaminasen dient; und
- den Analogen der Fragmente der Sequenz SEQ ID NO 2, die wenigstens eine 80-prozentige Homologie mit einem der Fragmente der SEQ ID NO 2 aufweisen, wobei die Analoge wenigstens eine Glutamin-Domäne (Gln) aufweisen - wobei Lysin (Lys) als Substrat für die Transglutaminasen dient,
zur Anwendung in der Behandlung von Hyperkeratose, Xerose, Ichthyosen, Psoriasis, benignen oder malignen hyperkeratotischen tumorösen Läsionen oder reaktionsbedingten Keratosen.

6. Anwendung nach Anspruch 1 oder 2 oder Polypeptid nach einem der Ansprüche 3 bis 5, **dadurch gekennzeichnet, dass** das Polypeptid ein Fragment mit einer Länge von wenigstens 7, bevorzugt wenigstens 9 und insbesondere wenigstens 15 aufeinanderfolgenden Aminosäuren ist.

7. Anwendung nach Anspruch 1 oder 2 oder Polypeptid nach einem der Ansprüche 3 bis 5, **dadurch gekennzeichnet, dass** das Polypeptid eine Sequenz umfasst, welche gewählt ist aus SEQ ID NO 3, SEQ ID NO 4, SEQ ID NO 5 und ihren Mischungen.

## Claims

1. A cosmetic use of an effective amount of a natural or synthetic isolated polypeptide belonging to the family of late proteins of the cornified envelope (LCE), said polypeptide being selected from:
- fragments of the sequence SEQ ID N°2 having a length of at least 5 consecutive amino acids, said fragments having at least one glutamine (Gln) - lysine (Lys) domain serving as a substrate for the transglutaminases; and
- analogs of said fragments of the sequence SEQ ID N°2 having at least 80% homology with one of said fragments of the sequence SEQ ID N°2, said analogs having at least one glutamine (Gln) - lysine (Lys) domain serving as a substrate for the transglutaminases,
for preventing and/or treating the signs of dry skin.

2. A cosmetic use of an effective amount of a natural or synthetic isolated polypeptide belonging to the family of late proteins of the cornified envelope (LCE), said polypeptide being selected from:
- fragments of the sequence SEQ ID N°2 having a length of at least 5 consecutive amino acids, said fragments having at least one glutamine (Gln) - lysine (Lys) domain of the sequence SEQ ID N°2, said fragments having at least one glutamine (Gln) - lysine (Lys) domain serving as a substrate for the transglutaminases; and
- analogs of said fragments of the sequence SEQ ID N°2 having at least 80% homology with one of said fragments of the sequence SEQ ID N°2, said analogs having at least one glutamine (Gln) - lysine (Lys) domain serving as a substrate for the transglutaminases,
for preventing and/or treating the signs of skin aging.

3. A natural or synthetic isolated polypeptide belonging to the family of late proteins of the cornified envelope (LCE), said polypeptide being selected from:
- fragments of the sequence SEQ ID N°2 having a length of at least 5 consecutive amino acids, said fragments having at least one glutamine (Gln) - lysine (Lys) domain of the sequence SEQ ID N°2, said fragments having at least one glutamine (Gln) - lysine (Lys) domain serving as a substrate for the transglutaminases; and
- analogs of said fragments of the sequence SEQ ID N°2 having at least 80% homology with one of said fragments of the sequence SEQ ID N°2, said analogs having at least one glutamine (Gln) - lysine (Lys) domain serving as a substrate for the transglutaminases,
for use for preventing and/or treating trophic skin disorders or those following disorders of healing.

4. A natural or synthetic isolated polypeptide belonging to the family of late proteins of the cornified envelope (LCE), said polypeptide being selected from:
- fragments of the sequence SEQ ID N°2 having a length of at least 5 consecutive amino acids, said fragments having at least one glutamine (Gln) - lysine (Lys) domain of the sequence SEQ ID N°2, said fragments having at least one glutamine (Gln) - lysine (Lys) domain serving as a substrate for the transglutaminases; and
- analogs of said fragments of the sequence SEQ ID N°2 having at least 80% homology with one of said fragments of the sequence SEQ ID N°2, said analogs having at least one glutamine (Gln) - lysine (Lys) domain serving as a substrate for the transglutaminases,
for use for preventing and/or treating thinning of the epidermis and in particular of the stratum corneum and/or for treating excessive fragility of the skin and/or for inducing thickening of the stratum corneum.

5. A natural or synthetic isolated polypeptide belonging to the family of late proteins of the cornified envelope (LCE), said polypeptide being selected from:
- fragments of the sequence SEQ ID N°2 having a length of at least 5 consecutive amino acids, said fragments having at least one glutamine (Gln) - lysine (Lys) domain of the sequence SEQ ID N°2, said fragments having at least one glutamine (Gln) - lysine (Lys) domain serving as a substrate for the transglutaminases; and
- analogs of said fragments of the sequence SEQ ID N°2 having at least 80% homology with one of said fragments of the sequence SEQ ID N°2, said analogs having at least one glutamine (Gln) - lysine (Lys) domain serving as a substrate for the transglutaminases,
for use for treating hyperkeratosis, xerosis, ichthyoses, psoriasis, benign or malignant hyperkeratotic tumoral lesions or reactive keratoses.

6. The use according to claim 1 or 2, or the polypeptide according to any one of 3 to 5, **characterized in that** the polypeptide is a fragment with a length of at least 7, preferably at least 9, and more particularly at least 15 consecutive amino acids.

7. The use according to claim 1 or 2, or the polypeptide according to any one of 3 to 5, **characterized in that** the polypeptide has a sequence selected from SEQ ID N°3, SEQ ID N°4, SEQ ID N°5, and mixtures thereof.
